# EUROPEAN PATENT SPECIFICATION

(11) **EP 4 165 107 B1**
(45) Date of publication and mention of the grant of the patent: **07.02.2024**
(21) Application number: 21730611.7
(22) Date of filing: 08.06.2021
(51) Int. Cl.: C08G 65/48, C08G 73/02, C07D 265/16

(54) **BENZOXAZINE DERIVATIVES VITRIMERS**
BENZOXAZINDERIVATE ALS VITRIMERE
DÉRIVÉS DE BENZOXAZINE EN TANT QUE VITRIMÈRES

(30) Priority: 10.06.2020 LU 101846
(43) Date of publication of application: 19.04.2023
(73) Proprietor: Luxembourg Institute of Science and Technology (LIST), 4362 Esch-sur-Alzette (LU)
(72) Inventor: ADJAOUD, Antoine, 4362 Esch-sur-Alzette (LU); PUCHOT, Laura, 4362 Esch-sur-Alzette (LU); TREJO MACHIN, Acerina, 4362 Esch-sur-Alzette (LU); VERGE, Pierre, 4362 Esch-sur-Alzette (LU)
(74) Representative: Lecomte & Partners
(86) International application number: PCT/EP2021/065335
(87) International publication number: WO 2021/250024

(56) References cited:
- RAO B S ET AL: "Synthesis and characterization of difunctional benzoxazines from aromatic diester diamine containing varying length of aliphatic spacer group: Polymerization, thermal and viscoelastic characteristics", EUROPEAN POLYMER JOURNAL, PERGAMON PRESS LTD OXFORD, GB, vol. 77, 3 February 2016 (2016-02-03), pages 139-154, XP029443899, ISSN: 0014-3057, DOI: 10.1016/J.EURPOLYMJ.2016.02.003
- TREJO-MACHIN ACERINA ET AL: "Phloretic acid as an alternative to the phenolation of aliphatic hydroxyls for the elaboration of polybenzoxazine", GREEN CHEMISTRY, vol. 19, no. 21, 18 September 2017 (2017-09-18), pages 5065-5073, XP055775311, GB ISSN: 1463-9262, DOI: 10.1039/C7GC02348K
- KISKAN BARIS ET AL: "Synthesis, characterization, and properties of new thermally curable polyetheresters containing benzoxazine moieties in the main chain : New Thermally Curable Polyetheresters", JOURNAL OF POLYMER SCIENCE PART A: POLYMER CHEMISTRY, vol. 46, no. 2, 4 December 2007 (2007-12-04), pages 414-420, XP055775301, US ISSN: 0887-624X, DOI: 10.1002/pola.22392
- MUSTAFA ARSLAN ET AL.: "Recycling and Self-Healing of Polybenzoxazines with Dynamic Sulfide Linkages", SCIENTIFIC REPORTS, vol. 7, 12 July 2017 (2017-07-12), page 5207, XP055725832, DOI: 10.1038/s41598-017-05608-2
- LEIBLER L. ET AL.: "Silica-Like Malleable Materials from Permanent Organic Networks", SCIENCE, vol. 334, 18 November 2011 (2011-11-18), pages 965-968, XP055775404,

## Description

The invention is directed to the field of ester-containing benzoxazine derivatives vitrimers and to a process of manufacturing thereof and the use of said vitrimers in various applications.

### Technical field

Composites are almost all the cases produced from thermoset resins, a material of choice for numerous applications because of their dimensional stability, mechanical properties and creep/chemical resistance. However, as a result of their permanent molecular architecture, they are impossible to recycle or to reprocess, and ends up in landfills.

A chemical way to tackle this drawback is offered by the introduction of exchangeable chemical bonds, leading to dynamic cross-links. Polymer networks containing such exchangeable bonds are also known as covalent adaptable networks (CANs) (W. Denissen et al. - Wim Denissen, Johan M. Winne and Filip E. Du Prez, Chem. Sci., 2016, 7, 30-38). CANs may be further classified into two groups depending on their exchange mechanism, either dissociative or associative. In the first, chemical bonds are first broken and then formed again at another place. Diels Alder reactions are the most common mechanism of dissociative CANs. In the second, polymer networks do not depolymerise upon heating, but are characterized by a fixed cross-link density. Covalent bonds are only broken when new ones are formed, making these networks permanent as well as dynamic. The first reported associative CANs (2005) were based on photo-mediated reactions by using allyl sulfides for instance. Later, a similar exchange mechanism was introduced by using alternative radical generators with trithiocarbonates. In 2011, Leibler *et al.* (D. Montarnal, M. Capelot, F. Tournilhac and L. Leibler, Science, 2011, 334, 965-968) extended the field of associative CANs by adding a suitable transesterification catalyst to epoxy/acid or epoxy/anhydride polyester-based networks, resulting in permanent polyester/polyol networks that show a gradual viscosity decrease upon heating. Such a distinctive feature of vitreous silica had never been observed in organic polymer materials. Hence, the authors introduced the name vitrimers for those materials.

Vitrimers are portrayed as the third class of polymeric material owing to their outstanding features. The dynamic nature of the covalent network, arises from reversible chemical bonds, allows the material to be healed, recycled and reprocessed like thermoplastics. These exchange reactions are triggered by external stimulus, most frequently temperature. The viscosity of vitrimers gradually decreased upon heating providing malleability to the network while permitting internal stress to relax. Network integrity over the entire range of application ensures mechanical and solvent resistance.

Following the prototypal vitrimer developed by Leibler *et al.* in 2011 (previously mentioned), dynamic transesterification reactions demonstrated extensive interest over the last decade. These chemical exchanges induced at elevated temperatures between ester linkages and hydroxyl groups are responsible for topology rearrangements. Transesterification mechanism was implemented in cross-linked network to design self-healable, recyclable and reprocessable material with tunable properties.

Demongeot et al. (A. Demongeot, R. Groote, H. Goossens, T. Hoeks, F. Tournilhac and L. Leibler, Macromolecules, 2017, 50 (16), 6117-6127) adapted the vitrimer concept to commercially available thermoplastic. Cross-linked polybutylene terephthalate (PBT) vitrimer based on transesterification exchanges was successfully prepared by reactive extrusion. In addition to improving the manufacturing techniques and the potential scope of these networks, global environmental context urges the scientific community to promote sustainable polymer derived from naturally occurring feedstocks. Altuna et al. (F. I. Altuna, V. Pettarin and R. Williams, Green Chem., 2013, 15, 3360-3366) endeavoured to generate fully bio-based polyester showing properties reminiscent of vitrimers, starting from epoxidized soybean oil and an aqueous citric acid solution. Furthermore, Legrand et al. (A. Legrand and C. Soulié-Ziakovic, Macromolecules, 2016, 49, 5893-5902) enabled to extend the scalability of applications of vitrimer networks by developing a silica-reinforced epoxy vitrimer nanocomposites with enhanced properties.

Polybenzoxazines are a new type of thermoset with outstanding mechanical and thermal properties. As many other thermosets, they cannot be reshaped, re-processed nor recycled. A few examples have been reported showing a reasonable level of healability (L. Zhang, Z. Zhao, Z. Dai, L. Xu, F. Fu, T. Endo, X. Liu, ACS Macro. Lett. 2019, 8, 5, 506-511 and Arslan M., Kiskan B., Y. Yagci, Sci. Rep. 2017, 7, 5207). However, polybenzoxazine remains a class of high performance materials without any demonstration of vitrimers capabilities. Such sustainable vitrimer will widespread the use of polybenzoxazine towards smart coatings, reversible adhesives, or even recyclable matrix resins for composite materials.

### Disclosure of the invention

The invention has for technical problem to provide a solution to at least one drawback of the above cited prior art.

For this purpose, the invention is directed to an ester containing benzoxazine monomer of formula (I) 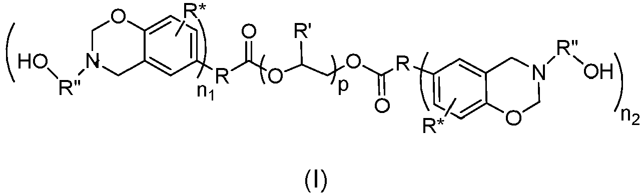 wherein, independently,
- at least one R* group is present in the benzoxazine cycle, and is selected from the group consisting of H, an aliphatic C₁-C₆ alkyl group, OH, an aliphatic C₁-C₆ alkoxy group, an aliphatic C₂-C₆ alkenyl group, an aliphatic C₁-C₆ alkyl or alkoxy substituted or unsubstituted phenyl group, 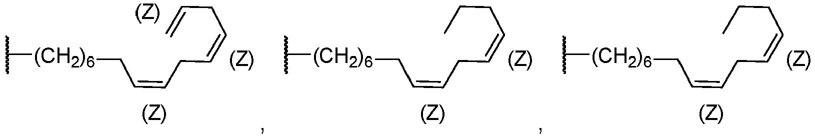 and 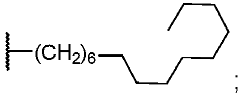
- R is either selected from the group consisting of an aliphatic C₁-C₆ alkyl group, an aliphatic C₁-C₆ alkyl or alkoxy substituted or unsubstituted phenyl group, a C₂-C₆ alkenyl group, -(CH₂)ₙ₃- wherein n₃ is an integer from 1 to 10, - CH(aliphatic C₁-C₆ alkyl group), -CH(aliphatic C₁-C₆ alkyl or alkoxy substituted or unsubstituted phenyl group), or R is omitted;
- R' is selected from the group consisting of H, -(CH₂)ₙ₃-OH, and 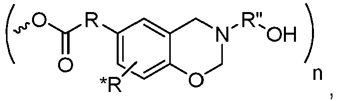 wherein n = n₁ = n₂ and are, independently, an integer of from 1 to 3, and R, the at least one R* and n₃ are as defined above;
- R" is an aliphatic C₁-C₆ alkyl group; and
- p is an integer of from 1 to 50.

The ester-containing benzoxazine monomer of the invention is advantageously suited for obtaining polybenzoxazine derivatives vitrimers by a polymerization involving the benzoxazine ring opening and a self-polymerisation under heat, resulting to said polybenzoxazine derivatives vitrimers. Owing to the specific monomer starting product, the vitrimers of the invention exhibit self-healing, reshaping, reprocessability and recycling properties. For the rest of the document, benzoxazine vitrimers will always refer to the polymerized form of the ester-bond benzoxazine monomers. "Derivative" means any compound which derives from the benzoxazine structure and which may have some various moieties or groups not modifying the base structure.

The polybenzoxazine derivatives vitrimers properties are tightly connected to the properties of the ester-containing benzoxazine monomer.

As may be seen from formula (I), the monomer includes a benzoxazine ring moiety that allows the cross-linking of said monomer upon heating and that promotes the reprocessing of the obtained benzoxazine vitrimers thanks to the exchangeable ester bonds it forms once crosslinked. Benzoxazine gives thermosetting properties such as high-temperature and flammability performance, high strength, thermal stability, low water absorption, chemical resistance, low melt viscosities, and near-zero shrinkage.

The presence of a moiety consisting in ester bonds and free aliphatic hydroxyl groups are essential to form a dynamic and reversible network of the benzoxazine derivatives vitrimers, allowing the material to be recycled, reshaped and reprocessed. An amine terminated with a hydroxyl group allows to close the oxazine ring and allows the transesterification reactions. Accordingly, the essential features of the monomer of the invention rely on the benzoxazine-containing moiety, ester bonds and free aliphatic hydroxyl groups. The Tg of such polybenzoxazine may be of from 25°C to 300°C.

Preferably, the p integer may be in the range of from 1 to 30, more preferably of from 1 to 20, most preferably of from 1 to 10, said ranges, independently, being selected for fine-tuning the processing temperature and relaxation of the benzoxazine vitrimers obtained through the polymerization of said monomers, and for better mechanical and thermic properties of the vitrimers.

In the context of the invention, "aliphatic" group is a linear or branched group.

At least one R* group, more preferably 1-3 R* group(s), may be present in the benzoxazine cycle, and the R* group is selected from the group consisting of H, an aliphatic C₁-C₄ alkyl group, OH, an aliphatic C₁-C₄ alkoxy group, 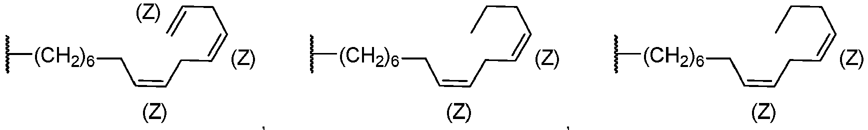 and 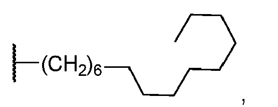
R may either be selected from the group consisting of an aliphatic C₁-C₃ alkyl group, an aliphatic C₁-C₃ alkyl or alkoxy substituted or unsubstituted phenyl group, a C₂-C₄ alkenyl group, -(CH₂)ₙ₃- wherein n₃ is an integer from 1 to 6, -CH(aliphatic C₁-C₃ alkyl group), -CH(aliphatic C₁-C₃ alkyl or alkoxy substituted or unsubstituted phenyl group), or R may be omitted;
R' is selected from the group consisting of H, -(CH₂)ₙ₃-OH, and 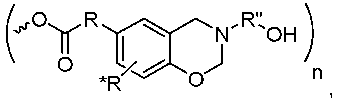 wherein n = n₁ = n₂ and are, independently, an integer of from 1 to 3, more preferably are 1 or 2, and R, R* and n₃ are as defined above.

The invention also relates to a process for synthesizing an ester-containing benzoxazine monomer of formula (I) comprising the following steps consisting of:
a) reacting a phenolic acid derivative of formula (II), comprising at least one R* group, 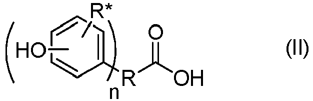 with a polyfunctional molecule or oligomer of formula (III) 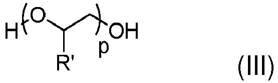 at a temperature of from 25°C to 200°C, during 1h-72h, in the presence of a catalyst of Bronsted acid type, resulting in a phenol terminated oligomer or molecule of formula (IV) 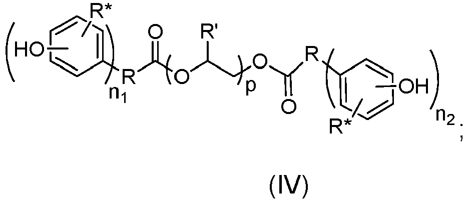 and
b) reacting the compound of formula (IV) with a mixture of:
   - an amino-alcohol bifunctional derivative of formula (V): 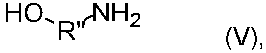 and
   - an aldehyde derivative
   at a temperature range of from 25°C to 100°C, during 0,5h to 48h, wherein R, R', R", the at least one R*, n, n₁, n₂, p are, independently, as defined above, with the proviso that when the at least one R* of the phenolic acid derivative is in *ortho* position with regard to -OH group, then R* is H.

The ester-containing benzoxazine monomer of the invention is advantageously suited for obtaining polybenzoxazine derivatives vitrimers by a polymerization involving the benzoxazine ring opening and a self-polymerisation under heat.

The Applicant has shown that the specific starting reactants are providing an ester-containing benzoxazine monomer, which in turn, after polymerization, is giving the polybenzoxazine derivatives vitrimers comprising polymerized benzoxazine.

"Derivative" in the expressions "phenolic acid derivative", "amino-alcohol bifunctional derivative" and "aldehyde derivative" means any compound which respectively has/bears a phenolic acid, an amino-alcohol bifunctional and aldehyde base structure.

The benzoxazine ring, obtained from the reaction of the specific derivatives (formulae (II)-(V)) which allows the material to be cross-linked (processed) upon heating, helps the reprocessing thanks to the exchangeable and reversible ester bonds, and free aliphatic hydroxyl groups. Also, the benzoxazine ring moiety gives thermosetting properties such as high-temperature and flammability performance, high strength, thermal stability, low water absorption, chemical resistance, low melt viscosities, and near-zero shrinkage.

Accordingly, the first step of ester-containing benzoxazine monomer synthesis (step a)) typically corresponds to a Fischer esterification between a polyfunctional molecule or oligomer (ditelechelic), terminated with aliphatic hydroxyl group, of formula (III), and a phenolic acid derivative of formula (II) in presence of a Bronsted acid type catalyst which may be introduced in catalytic amount.

The phenolic acid derivative (formula (II)) may include at least one R* group, more preferably of from 1 to 3, related to the substitution of the phenolic ring, and the R group related to the nature of the bridge between the ester bonds and the phenolic ring.

It is advantageous that the phenolic acid derivative (formula (II)) bears R* groups that does not interfere with the phenolic ortho-position to avoid steric hindrance that may adversely impact the kinetic of step a) or the oxazine ring closure of step b). Accordingly, R* groups may then be short chain groups with the proviso that R* in phenolic *ortho*-position is H.

In some embodiments, there could be two phenolic ortho-positions, each of which is H for the R* group.

The phenolic acid derivative may preferably be an aliphatic or an aromatic phenolic acid, or combination thereof.

The phenolic acid derivative may be more preferably selected from the group consisting of mono-, di-, tri-hydroxybenzoic acid derivatives, anacardic acid derivatives, hydroxycinnamic acid derivatives, aliphatic X-hydroxyphenyl acid derivatives, wherein X is 2-4, aliphatic diphenolic acid derivatives and triphenolic acid derivatives, or mixtures thereof. However, the triphenolic acids are the less preferred due to the steric hindrance.

Most preferred aliphatic mono-, di-, tri-hydroxybenzoic acid derivatives may be of formula (VI) 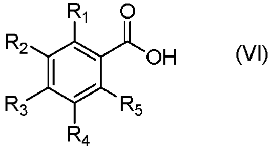 wherein R is omitted, and at least one of R₁ to R₅ corresponds to R*, and at least one among R₁-R₅ is selected from the group consisting of 1, 2 and 3 hydroxyl group(s), then at least one H is in phenolic ortho-position, the rest being at least one of H and an aliphatic alkyl group of C₁-C₆.

Especially, in formula (VI), at least one combination of R₁ to R₅ may be selected from the group consisting of:
R₁= OH, R₂=H, R₃=R₄=R₅= H or CH₃ or CH₂-CH₃ or CH₂-CH₂CH₃ or CH₂-CH(CH₃)₂,
R₂= OH, R₁=R₃=H, R₄=R₅= H or CH₃ or CH₂-CH₃ or CH₂-CH₂CH₃ or CH₂-CH(CH₃)₂,
R₃= OH, R₂=R₄=H, R₁=R₅= H or CH₃ or CH₂-CH₃ or CH₂-CH₂CH₃ or CH₂-CH(CH₃)₂,
R₄= OH, R₃=R₅= H, R₁=R₂= H or CH₃ or CH₂-CH₃ or CH₂-CH₂CH₃ or CH₂-CH(CH₃)₂,
R₁=R₂= OH, R₃=H, R₄=R₅= H or CH₃ or CH₂-CH₃ or CH₂-CH₂CH₃ or CH₂-CH(CH₃)₂,
R₁=R₃= OH, R₂=R₄= H, R₅ = H or CH₃ or CH₂-CH₃ or CH₂-CH₂CH₃ or CH₂-CH(CH₃)₂,
R₁=R₄= OH, R₂=R₃= R₅= H
R₁=R₅= OH, R₂=R₄= H, R₃=H or CH₃ or CH₂-CH₃ or CH₂-CH₂CH₃ or CH₂-CH(CH₃)₂,
R₂=R₃= OH, R₁=R₄=H, R₅= H or CH₃ or CH₂-CH₃ or CH₂-CH₂CH₃ or CH₂-CH(CH₃)₂,
R₂=R₄=OH, R₁=R₃=R₅= H
R₁=R₃=R₅= OH, R2=R4= H,
   and
R₂=R₃=R₄= OH, R₁=R₅= H.

Most preferred anacardic acid derivatives may be of formula (VII), wherein R₆=R* 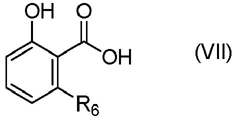 wherein R is omitted, and R₆ is

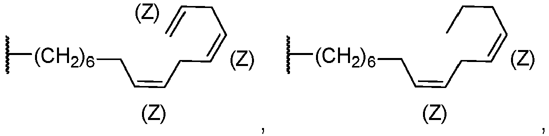

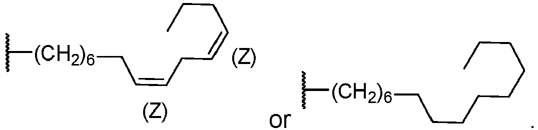

Most preferred hydroxycinnamic acid derivatives may be of formula (VIII) 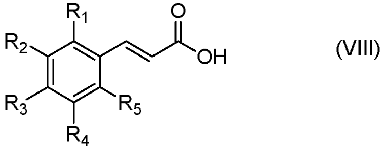 wherein at least one of R₁ to R₅ corresponds to R*, and at least one among R₁-R₅ may be selected from the group consisting of 1 and 2 hydroxyl group(s) and at least one H being in phenolic ortho-position, the rest being at least one of H and an aliphatic alkyl or alkoxy group of C₁-C₆.

Especially, in formula (VIII), at least one combination of R₁ to R₅ may be selected from the group consisting of:
R₅= OH, R₄= H, R₁=R₂=R₃= H or CH₃ or CH₂-CH₃ or CH₂-CH₂CH₃ or CH₂-CH(CH₃)₂,
R₄= OH, R₃=R₅= H, R₁=R₂= H or CH₃ or CH₂-CH₃ or CH₂-CH₂CH₃ or CH₂-CH(CH₃)₂,
R₃= OH, R₂=R₄= H, R₁=R₅= H or CH₃ or CH₂-CH₃ or CH₂-CH₂CH₃ or CH₂-CH(CH₃)₂,
R₃= OH, R₂= O(C₁-C₆ alkyl group), R₁=R₄=R₅= H, and
R₂= R₃= OH, R₁=R₄= H, R₅= H or CH₃ or CH₂-CH₃ or CH₂-CH₂CH₃ or CH₂-CH(CH₃)₂.

Most preferred aliphatic X-hydroxyphenyl acid derivatives may be selected from the group consisting of aliphatic di-hydroxyphenyl acids (X=2), aliphatic tri-hydroxyphenyl acids (X=3) and aliphatic tetra-hydroxyphenyl acids (X=4), or mixtures thereof, of formula (IX) 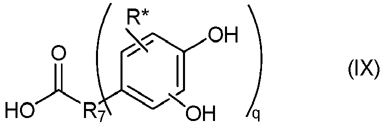 wherein
- R₇, corresponding to R, independently of the nature of X-hydroxyphenyl aliphatic acid derivatives, is selected from the group consisting of (CH₂)ₙ₄, CH(CH₂)ₙ₅-(aliphatic C₁-C₆ alkyl or alkoxy substituted or unsubstituted phenyl group), wherein n₄ is an integer from 1 to 12, preferably from 1 to 10, n₅ is an integer from 0 to 12, preferably from 0 to 10, CH(CH₂)ₙ₅(CH₃), CH(CH(CH₃)₂), C(CH₃)₂, CH(aliphatic C₁-C₆ alkyl or alkoxy substituted or unsubstituted phenyl group);
- the number of R* in the ring is depending on the number of hydroxyl groups in the ring, and at least one R*, preferably of from 1 to 3, is H towards the phenolic ortho-position, and, independently, is selected from the group consisting of (CH₂)ₙ₄CH₃, (CH₂)ₙ₄-(aliphatic C₁-C₆ aliphatic alkyl or alkoxy substituted or unsubstituted phenyl group), wherein n₄ is an integer from 1 to 12, preferably from 1 to 10, more preferably 1-6, and (CH₂)ₙ₄(CH(CH₃)₂); and
- the integer q is comprised between 1 and 3.

When n₅ is 0, the (CH₂) group is omitted.

Most preferred diphenolic acid derivatives are of formula (X) 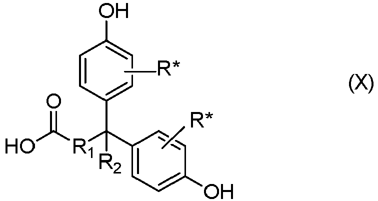 wherein
on each respective phenolic cycle, at least one R*, preferably of from 1 to 3, is H towards the phenolic ortho-position, and otherwise R* and R₂, independently, are selected from the group consisting of (CH₂)ₙ₄CH₃, (CH₂)ₙ₄-(aliphatic C₁-C₆ aliphatic alkyl or alkoxy substituted or unsubstituted phenyl group), wherein n₄ is an integer from 1 to 12, preferably from 1 to 10, more preferably from 1 to 6, and (CH₂)ₙ₄(CH(CH₃)₂), and
R₁ is selected from the group consisting of (CH₂)ₙ₅, wherein n₅ is an integer from 1 to 3, CH(CH₂)ₙ₅(CH₃), CH(CH(CH₃)₂) and C(CH₃)₂, (CH₂)ₙ₅ being the most preferred to lower the steric hindrance.

In said diphenolic acid derivatives, R= -R₁-C-R₂- moiety. Most preferred is the 4,4-Bis(4-hydroxyphenyl)valeric acid (VA).

The polyfunctional molecule or oligomer compound of formula (III) is of importance for selecting the processing temperature of the benzoxazine polymer.

The compound of formula (III) may advantageously have 1-30, better 1-20, especially 1-10, p values, and may represent more preferably, when R'=H, a polyethylene glycol (PEG) with a molecular weight (MW) in the range of from 4 MW of the C₂H₄O unit to 50 MW of the C₂H₄O unit, the MW of the C₂H₄O unit being classically of about 44,05 g/Mol. It is preferable to use commercially available PEG, for example PEG 200 to PEG 2200, as being easily available.

In the compound of formula (III), when R'=H, p values may be of from 1 (ethylene glycol) to 3 (triethylene glycol -TEG).

In some other embodiments, the compound of formula (III) may be glycerol (R'=CH₂OH).

The Bronsted acid type catalyst are those commonly used for a Fischer esterification include para-toluene sulfonic acid (APTS), anhydrous chlorhydric acid (HCl), phosphoric acid (H₃PO₄), methanoic acid (CH₃-CO₂H), sulfuric acid, tosylic acid, and Lewis acids such as scandium(III) triflate. The content of catalyst is of from 0,5 wt% to 2 wt%.

The step a) may advantageously be carried out at a temperature in the range of 60°C to 150°C, most preferably of from 100°C to 140°C for the best synthesis yields of higher than 95%, the chosen temperature being dependent on the nature of the reactants, i.e. the melting temperature of said reactant medium.

Advantageously, step a) is performed of from 12h to 48h for the highest yield of at least 95%, and the duration is based on the kinetic of the reaction.

The respective stoichiometry of starting reactants on step a), phenolic acid derivative:polyfonctional molecule or oligomer may preferably be 1,0-3,0 eq.:1,0 eq, resulting in an 1,0 eq. of phenol terminated oligomer or molecule.

The second step of the process, step b), corresponds to a Mannich condensation type reaction of the phenol terminated oligomer or molecule of step a) (formula IV) with an amino-alcohol bifunctional derivative (formula (V)) and an aldehyde derivative, optionally in presence of a catalyst. Thus, since step b) does not use a catalyst, step b) is implemented in an easier way.

Advantageously, the amino-alcohol bifunctional derivative of formula (V) includes a linear amino-alcohol derivative with a primary amine moiety and an aliphatic hydroxyl moiety for obtaining with the highest yield and the best reaction conditions the oxazine ring.

The amino-alcohol bifunctional derivative of formula (V) may more preferably be selected from the group consisting of 2-aminoethanol, 2-amino-2-methylpropanol, 5-aminopentan-1-ol, heptaminol and diglycolamine.

Preferably, the aldehyde derivative is selected from the group consisting of formaldehyde, paraformaldehyde of formula 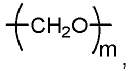 where m is an integer of from 8 to 100,
acetaldehyde, propionaldehyde, butylaldehyde, polyoxymethylene and aldehydes having the general formula R₉CHO, where R₉ is a substituted or unsubstituted aliphatic C₁-C₂₀ alkyl group optionally containing heteroatoms, or mixtures thereof.

In the aldehyde derivative, R₉ may preferably be a substituted or unsubstituted aliphatic C₁-C₁₅ alkyl group optionally containing heteroatoms, such as N, O, S, more preferably R' may be a substituted or unsubstituted aliphatic C₁-C₈ alkyl group optionally containing said optional heteroatoms.

The temperature range of step b) may preferably be of from 75°C to 100°C, more preferably of from 75°C to 95°C allowing to obtain the highest conversion yields of at least 95%.

Advantageously, step b) is performed from 1h to 12h, most preferably of from 2h to 4h for the highest yield of at least 95%.

One advantage of the invention, is that step b) is performed without any catalyst.

However, several catalysts can be used to catalyze the transesterification reaction (step b)), said catalysts being selected from the group consisting of Zn(II)(R₁₀)₂ wherein R₁₀ may preferentially be Cl⁻, CH₃CO₂⁻, CH₃-C(=O)-O⁻, CH₃COCHCOCH₃⁻, CH₃(CH₂)_{r:1-15}CH₂CO₂⁻; triazobicyclodecene (TBD); triphenylphosphine (PPh₃) and para-toluene sulfonic acid (APTS). It should be noticed that the presence of said catalyst slightly provided a better char yield (flame resistance) of the obtained benzoxazine monomer. This enhanced char yield may be of from 15% to 30%. The content of catalyst may be of from 0,5 wt% to 2 wt%.

The respective stoichiometry of starting reactants on step b), phenol terminated oligomer or molecule: amino-alcohol bifunctional derivative: aldehyde derivative may preferably be 1,0 eq.:1,0-18,0 eq:2,0-36,0 eq, resulting in an 1,0 eq. of the ester-containing benzoxazine monomer.

The specific range stoichiometry is depending on the respective functionality degree of the amino-alcohol bifunctional derivative and of the aldehyde derivative. Besides, the selected stoichiometry ranges of both amino-alcohol bifunctional derivative and aldehyde derivative preferably avoids the formation of either reaction linear and/or aliphatic by-products, such as oxazolidine, triaza derivatives, or condensation derivatives.

Preferentially, the whole process is performed with bio-based reactants.

The monomer synthesis may most preferably be solventless, even though a solvent could be added for the dissolution of starting reactants. The process involves a one-step synthesis, which is one of the advantages of the invention.

Advantageously, the whole synthesis may generally not require any further monomer purification for the invention to be implemented. However, the purification of the monomer, if needed, may be performed by any known technic (vacuum, distillation etc.)

The reaction mixtures of both steps a) and b) are stirred using a classical mechanical stirrer, or any non-limitative means.

The process may be implemented by any known means known to the one skilled in the art, using appropriate vessel either at lab scale or at industrial scale.

The invention also relates to a process for preparing a polybenzoxazine derivative vitrimer comprising the step of polymerization of an ester-containing benzoxazine monomer of the invention or as obtainable by the above mentioned process at temperatures within the range of from 100°C to 250°C for 1h to 24h, for obtaining polybenzoxazine derivatives vitrimers.

According to the process for preparing the vitrimers of the invention, the polymerization step, which is a curing step, allows the benzoxazine ring to open and to react on itself to form a 3D network. Once cooled, the shape of the material is kept even after few months, typically 2- 4 months. Once re-heated to at least 100 °C for a few minutes, the ester bonds are exchanging with the aliphatic hydroxyl group allowing the material to be reshaped, recycled, or reprocessed; while keeping structural integrity and number of covalent bound. Considering that Mannich condensation reaction is quantitative, nearly two hydroxyls groups could react with each ester bound through transesterification reaction (even after curing). The vitrimer behaviour strongly depend on the vitrimer glass transition (Tᵥ) also considered as the temperature where the transesterification reaction significantly increased. The vitrimer behaviours were demonstrated through several experiments. After the curing step, by heating the vitrimer above the Tᵥ, an initial shape of the vitrimer can be designed to other original shape. For example, vitrimers may be ground to a powder and can be reshaped or reprocessed at 150 °C in a couple of minutes. However its shape remains stable at room temperature.

The polymerization duration is depending on the curing temperature and/or on the nature of the ester-containing benzoxazine monomer. The polymerization temperature is selected for a given monomer to be higher than the temperature needed to synthesize the monomer. Generally, the higher the polymerization temperature, the shorter the curing duration. For example, when the temperature of the polymerization is 250°C, the curing duration may be of at least 1h, and for a polymerization temperature of 100°C, the curing duration may be of no more than 24h. Preferably, the curing temperature may be of from 140°C to 200°C, more preferably of from 140°C to 180°C, the latter range providing curing duration of from 1,5h to 3h, preferably of from 1,5h to 2,5h. The polymerization may be performed by any known heating means, such as laser beam and infrared beam.

The process may also include a post-polymerization step consisting of a heating step which may preferably be carried out at higher temperature than that the polymerization heating step.

The invention is also directed to a polybenzoxazine derivative vitrimer, that may be obtained by the above depicted process, exhibiting at least one of the following characteristics:
(i) Tᵥ values of from 120°C to 220°C; preferably of from 150°C to 200°C, more preferably of from 150°C to 170°C, and
(ii) Relaxation temperature values, ≥ Tᵥ values, of from 120°C to 270°C, preferably of from 150°C to 200°C, more preferably of from 150°C to 180°C.

The vitrimers Tᵥ values are generally dependent from the nature and the content of the catalyst of step b), when present.

The relaxation temperatures typically correspond to the relaxation temperatures of the vitrimers after the appliance of a strain, for example a physical deformation such as a torsion, without the observation of vitrimers degradation.

Advantageously, the vitrimers may also exhibit at least one of the following characteristics selected from the group consisting of:
- a relaxation time of from 0,5 s to 2 h, preferably of from 1 s to 1 h, more preferably of from 5 s to 50 min. The relaxation time is conventionally defined as the time for the sample to relax to a value corresponding 1/e (0,37) of its original modulus Generally, the higher is the temperature, the shorter is the relaxation time. For example, the relaxation time is about 5 min-20 s at temperatures values of 120°C-150°C, and of ≤ 20, preferably 5 s-20 s, at temperature ranges of 150°C to 200°C.
   In some embodiments, the vitrimer may be deformed between 0,1% to 100% of its initial size;
- an activation energy related to relaxation times may be of from 50 kJ/mol to 200 kJ/mol, preferably of from 70 kJ/mol to 170 kJ/mol, more preferably of from 100 kJ/mol to 160 kJ/mol; and
- a processing temperature may be of from 100°C to 250°C, preferably of from 130°C to 250°C, more preferably of from 150°C to 200°C, most preferably of from 150°C to 170°C.

The vitrimers according to the invention may also very preferably exhibit the characteristics of behaving as a thermoset and/or an insolubility in many solvents, without been limited, such as water, CHCl₃, CH₂Cl₂, DMF, THF, aromatic solvents, such as toluene and/or xylene, ketones, alcohols or carboxylic acids. Swelling properties are observed as an extent of from 0 to 500% of the initial weight thereof. Swelling experiments may be carried out in various solvents, for example in acetone, chloroform and water to assess the formation of a cross-linked network. Among them, chloroform is one of the solvents in which the vitrimer may show the highest swelling ratio of about 100%. In acetone and water, some vitrimers swell of 40%-50% and 20%-30%, respectively. Some other vitrimers may show swelling properties in water of 150% to 230%.

The vitrimers of the invention present self-healing, reshaping, reprocessability, recycling and reversible adhesive properties.

The vitrimers may constitute an intermediate layer between at least two substrates, such as metal, polymer, glass and ceramic material. The resulting composite material may be prepared by setting at least one ester-containing benzoxazine monomer between the two considered substrates then curing at a temperature providing the vitrimer without altering the integrity of the substrates. Each substrate may be different from the other.

Metallic substrates are not limited, and may be of aluminium, iron, steel and the like.

Polymer substrates may be of polycarbonate, acrylic, polyamide, polyethylene or terephthalate.

Benzoxazine vitrimers may then be advantageously used in non-limited various fields of technologies, such electronics, aerospace, defense and automotive fields.

The invention also relates to a composition A comprising:
a) an ester-containing benzoxazine monomer of formula (I), and
b) at least one or more additional compounds of organic molecules types containing or not benzoxazine moieties.

Preferably, the organic molecules types may be polymers containing or not benzoxazine moieties.

The additional compound may be used to enhance the properties of either the monomer or the vitrimer (i.e. viscosity, mechanical and thermal properties), or both.

Polymers may be epoxy resins, bismaleimide resins, phenolic resins or benzoxazine resins, polyurethanes, polyamides, polyolefins, polyesters, rubbers. The ester-containing benzoxazine derivative of formula I may be used in a weight ratio from 0,1 to 80 % of the final composition.

The compound of formula I may be used to provide vitrimer properties to the above mentioned polymers (self-healing, reprocessing, etc.).

The invention also relates to a composition B comprising:
a) an ester-containing benzoxazine monomer of formula (I), and
b) a material selected from the group consisting of fillers, fibers, pigments, dyes, and plasticizer.

The additional compound may be used to enhance the properties of either the monomer or the vitrimer (i.e. viscosity, mechanical and thermal properties), or both.

The additional compound could be carbon fibers, glass fibers, clays, carbon black, silica, carbon nanotubes, graphene, any known means for the thermal or the mechanical reinforcement of composites.

The invention also concerns a use of the vitrimer according to the invention as a reversible adhesive, sealant, coating or encapsulating systems for substrates selected from the group consisting of a metal, polymer, glass and ceramic material. Preferably, the metal and the polymer are as above defined.

The invention also relates to a use of the vitrimer according to the invention in 3D printing processes or in additive manufacturing processes.

### Brief description of the drawings

Other features and advantages of the present invention will be readily understood from the following detailed description and drawings among them:
- Figure 1 shows a synthesis reaction of an ester-containing benzoxazine monomer from 4,4-Bis(4-hydroxyphenyl)valeric acid (DPA) as a phenolic acid derivative;
- Figure 2 shows a network for a vitrimer obtained through the curing of the valeric acid benzoxazine monomer (schematized form);
- Figure 3 is a NMR spectrum of the valeric acid derivative benzoxazine monomer (PEG-DPA-mea);
- Figure 4a) displays the DSC curve and Figure 4b) the TGA of valeric acid benzoxazine monomer;
- Figure 5 illustrates the ability of the vitrimer of Figure 2 to be reshaped and reprocessed;
- Figure 6a) displays a Dilatometry curve (dL/L₀ (%) vs temperature) of the vitrimer obtained through the curing of the valeric acid benzoxazine monomer, the latter obtained without the use of any catalyst or with the use of 2% Zn(OAc)₂ catalyst in step b) ; and Figure 6b) exhibits the mechanical properties of the vitrimer;
- Figure 7a) depicts shear stress relaxation experiments: normalized relaxation modulus as a function of time between 120°C and 170°C; Fig. 7b): Arrhenius plot of the measured relaxation times for the vitrimer of Figure 2.
- Figure 8a) and Figure 8b) are respectively displaying the NMR spectrum of the valeric acid benzoxazine monomers (PEG₂₀₀-DPA-mea and PEG₂₀₀₀-DPA-mea).
- Figure 9 displays the DSC curve of PEGₙ-DPA-mea ester-containing benzoxazine monomers (n = 200 and 2000).
- Figure 10 shows the Isothermal rheology monitoring of PEGₙ-DPA-mea ester-containing benzoxazine monomers (n = 200 and 2000).
- Figure 11 displays Dilatometry curves of PEGₙ-DPA-mea ester-containing benzoxazine monomers (n = 200 or 2000).
- Figure 12a) and 12b) are respectively showing Stress relaxation curves of poly(PEG₂₀₀-DPA-mea) and poly(PEG₂₀₀₀-DPA-mea) ester-containing benzoxazine vitrimers.
- Figure 13 shows the Arrhenius plot of poly(PEGn-PA-mea) ester-containing benzoxazine vitrimer.
- Figure 14 is displaying the NMR spectrum of PEG₄₀₀-PA-mea ester-containing benzoxazine monomer (PA: phloretic acid).
- Figure 15 displays DSC curve of PEG₄₀₀-PA-mea ester-containing benzoxazine monomer (PA: phloretic acid).
- Figure 16 shows Isothermal rheology monitoring of PEG₄₀₀-PA-mea ester-containing benzoxazine monomer (PA: phloretic acid).
- Figure 17a) Stress relaxation curves and b) Arrhenius plot of poly(PEG₄₀₀-PA-mea) ester-containing benzoxazine vitrimer (PA: phloretic acid).

### Example 1: synthesis of an ester-containing benzoxazine monomer from 4,4-Bis(4-hydroxyphenyl)valeric acid (DPA) as a phenolic acid derivative

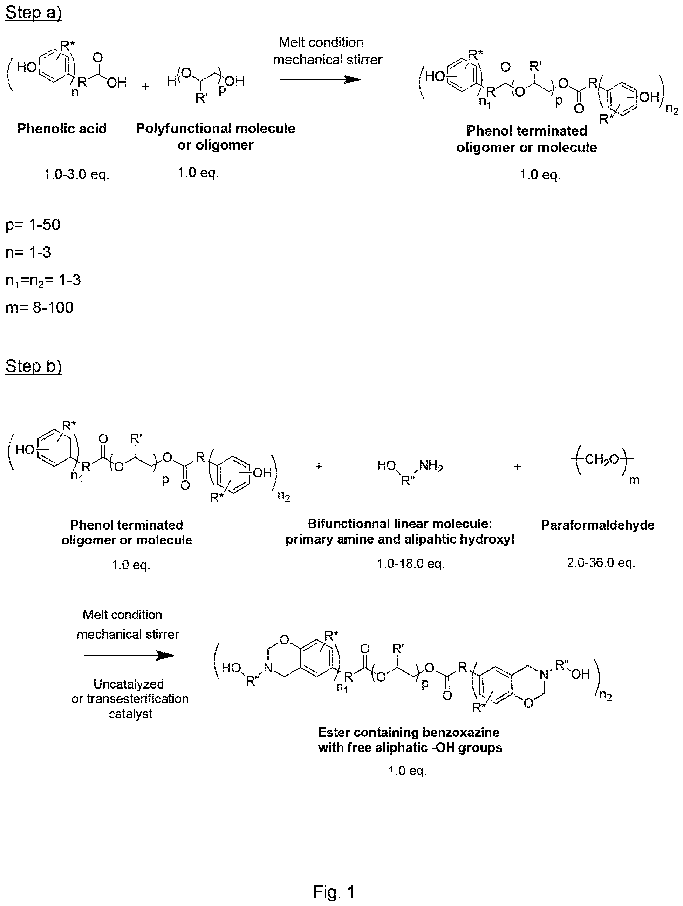

Ester-containing benzoxazine monomer was synthesized in two stages (Fig. 1).

The first step, step a), corresponds to a Fischer esterification between polyethylene glycol (PEG) (Mₙ = 400 g.mol⁻¹, p = 8-9, 1 eq, 10 g) and 4,4-Bis(4-hydroxyphenyl)valeric acid (DPA) (2 eq, 14,32 g) in presence of p-toluene sulfonic acid (pTSA) introduced in catalytic amount (1 wt%). PEG, DPA and pTSA were reacted together in melt at 130°C and agitated by mechanical stirring for 24 hours, to provide 4,4-Bis(4-hydroxyphenyl)valeric ester terminated polyethylene glycol (PEG-DPA).

The second step, step b), corresponds to a Mannich condensation between 4,4-Bis(4-hydroxyphenyl)valeric ester terminated polyethylene glycol (PEG-DPA) (1 eq, 22,8 g), ethanolamine (mea) (4 eq, 5,95 g) and paraformaldehyde (PFA) (8 eq, 5,84 g). In some examples, step b), is performed in presence of 2 wt% of Zn(OAc)₂ catalyst. All these reactants were reacted together in melt at 85°C and agitated by mechanical stirring for 2 hours to provide the ester-containing benzoxazine monomer named PEG-DPA-mea.

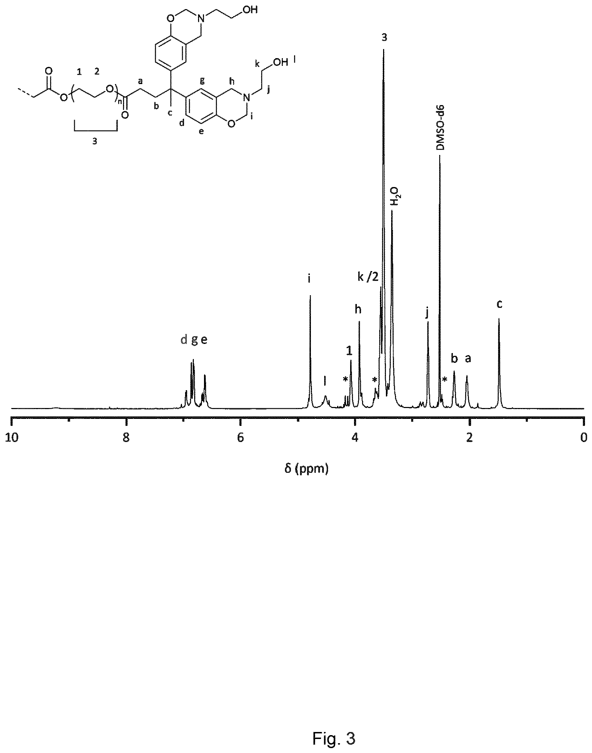

The Figure 3 is displaying the NMR spectrum (AVANCE III HD Bruker spectrometer) of PEG-DPA-mea ester-containing benzoxazine monomer synthesized in presence of 2 wt% of Zn(OAc)₂ catalyst in step b).

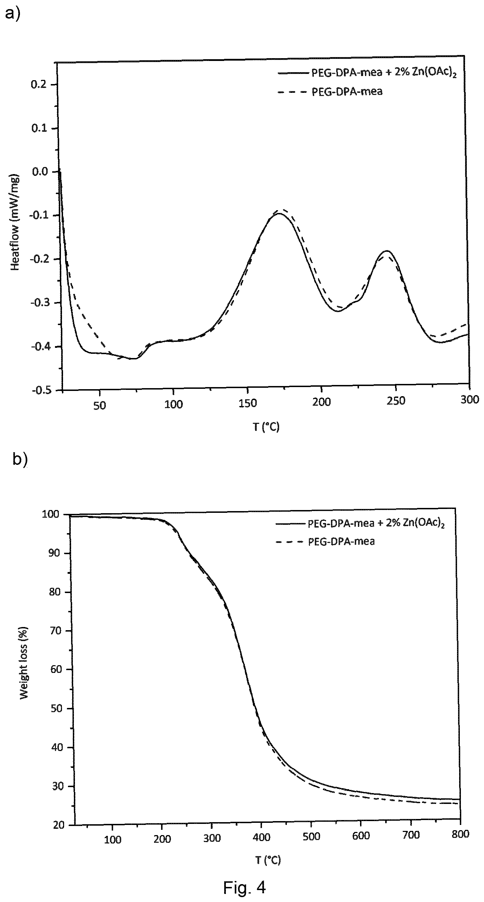

Figure 4a) and 4b) are respectively displaying the DSC and the TGA curves of the PEG-DPA-mea monomer in the presence (solid line) or in the absence (dash line) of Zn(OAc)₂ catalyst. Conditions: 10°C.min⁻¹, N₂ atmosphere.

The DSC curve (Fig. 4a) (Netzsch DSC 204 F1 Phoenix apparatus) shows an exothermic peak starting at a temperature of 105°C, with a maximum located at 174 °C. This peak corresponds to the ring opening of the benzoxazine rings upon heating. The second peak corresponds to the thermal decomposition of the ester linkage confirmed by TGA experiment (Fig. 4 b)(mass loss ≈ 6%). The second degradation stage is very similar to both samples with a weight loss of 46.7% and a maximum degradation temperature around T= 379°C. However, it should be noticed that the presence of catalyst slightly provided a better char yield (25,1%). At this stage, the material is therefore considered thermally stable up to at least 250°C (T_{d5%}).

### Example 2: synthesis of a vitrimer obtained through the curing of the PEG-DPA-mea monomer

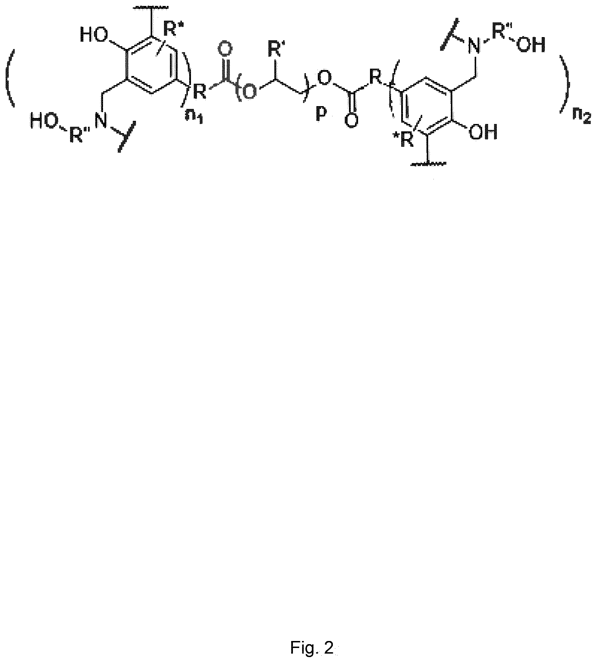

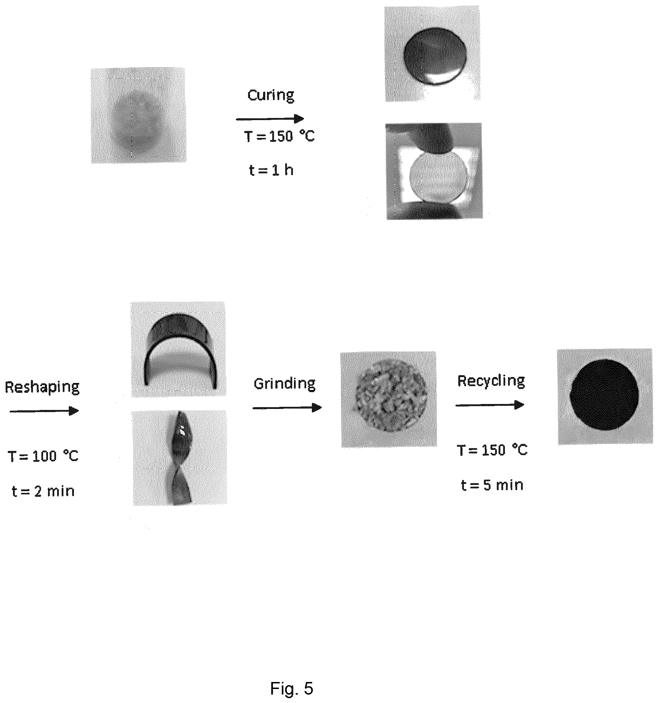

The benzoxazine monomer obtained in Example 1 was polymerized in a Teflon^{®} mold at 150°C during 1h, allowing the benzoxazine rings to open and to react on themselves to form a 3D network vitrimer (Fig. 2). Once cooled, the shape of the material is kept even after few months. Once re-heated to at least 100 °C for a few minutes, the ester bonds are exchanging with the aliphatic hydroxyl group allowing the material to be reshaped, recycled, or reprocessed; while keeping structural integrity and number of covalent bound. Considering that Mannich condensation reaction was quantitative, nearly two hydroxyls groups could react with each ester bound through transesterification reaction (even after curing). The vitrimer behaviour strongly depend on the vitrimer glass transition (Tᵥ) also considered as the temperature where the transesterification reaction significantly increased. The vitrimer behaviour of these samples was demonstrated through several experiments. After the curing step, by heating this material above the Tᵥ, the initial rod shape of the material can be designed to other original shape. Finally, the materials were ground to a powder and can be reshaped or reprocessed at 150 °C in a couple of minutes. However, its shape remains stable at room temperature as reported in Figure 5.

Swelling experiments were performed in acetone, chloroform and water to assess the formation of a cross-linked network of the vitrimer obtained through the curing of the PEG-DPA-mea monomer. Chloroform was the highest solvent in which the vitrimer showed the highest swelling ratio (≈ 100%). In acetone and water, vitrimers samples swell of 40 and 20%, respectively.

The material reacted with acetic acid to form an orange turbid suspension. The chemical decomposition of thermosets is an interesting recycling process.

Dilatometry experiments is a classical tool to reveal glass transition (T_{g}) and the vitrimer glass like transition (Tᵥ) of a vitrimer.

The device used is the Netzsch DIL 402 C apparatus with experimental conditions of 2 °C.min⁻¹, N₂ atmosphere.

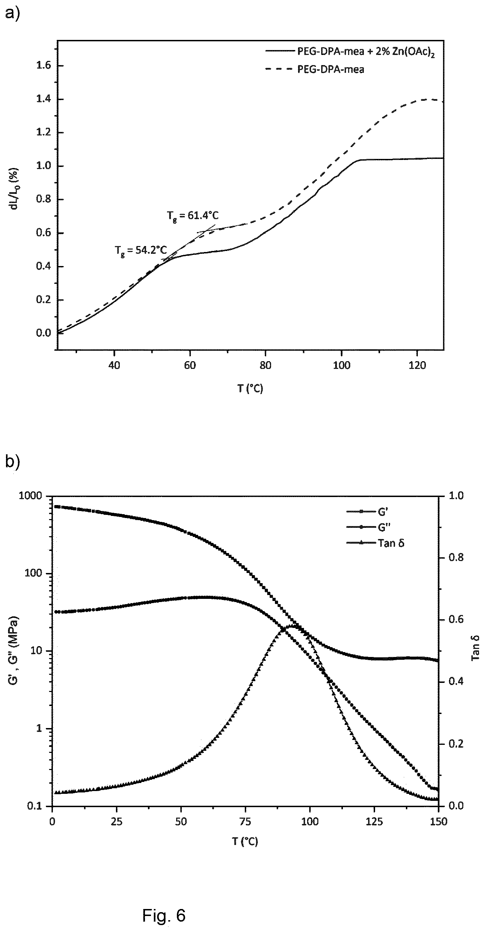

Two vitrimer samples were used, one obtained through the curing of PEG-DPA-mea monomer without the use of any catalyst in step b) (dash line) and the second one with the use of 2% Zn(OAc)₂ catalyst in step b) (solid line), results are depicted in Fig. 6. The plateau observed in for the catalyzed system is characteristic of the glass-like nature of the Tᵥ.

Mechanical properties were determined by rheological measurements recorded on Anton Paar Physica MCR 302 rheometer in rectangular-torsion mode with experimental conditions of γ= 0,1% constant deformation, f= 1Hz. The T_{g}s determined from the maximum in the loss modulus (G") and the maximum of the loss factor (tan δ) are 59 and 93 °C respectively.

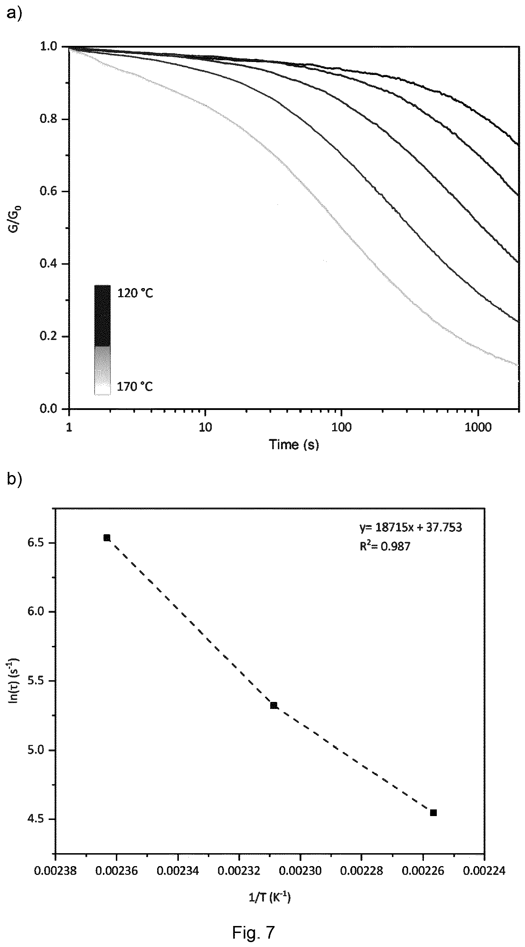

Viscoelastic properties of PEG-DPA-mea vitrimer were studied by stress relaxation experiments (Figure 7a). The relaxation time of the polymer was clearly noticeable and proportionally decreasing upon heating from 120°C (320 min) to 170°C (94 s).

The temperature dependence of the relaxation time is plotted in Figure 7b) following the Arrhenius law. The trend line in this plot fit to a thermally activated behavior for the relaxation time. The high correlation coefficient (R²= 0,987) means that these data are perfectly fitting the Arrhenius law. The activation energy from the Arrhenius equation was extracted using the slope of the trend line. The activation energy obtained from stress relaxation for PEG-DPA-mea vitrimer is 155 kJ.mol⁻¹.

### Example 3: synthesis of an ester-containing benzoxazine monomer from 4,4-Bis(4-hydroxyphenyl)valeric acid (DPA) as a phenolic acid derivative and different molecular weight of poly(ethylene glycol) (PEG) solutions

The first step, step a), corresponds to a Fischer esterification between polyethylene glycol (PEGₙ) (Mₙ = 200 or 2000 g.mol⁻¹ , p = 4-5 or 45-46 respectively, 1 eq, 10 g) and 4,4-Bis(4-hydroxyphenyl)valeric acid (DPA) (2 eq, 28.63 and 2.86 g, respectively for PEG₂₀₀ and PEG₂₀₀₀) in presence of p-toluene sulfonic acid (pTSA) introduced in catalytic amount (1 wt%). PEGₙ, DPA and pTSA were reacted together in melt at 130°C and agitated by mechanical stirring for 24 hours, to provide 4,4-Bis(4-hydroxyphenyl)valeric acid terminated polyethylene glycol (PEGₙ-DPA, wherein n = 200 or 2000).

The second step, step b), corresponds to a Mannich condensation between 4,4-Bis(4-hydroxyphenyl)valeric ester terminated polyethylene glycol (PEGₙ-DPA) (1 eq, 25 mmol, 18.2 or 63.1 g, respectively for PEG₂₀₀ and PEG₂₀₀₀), ethanolamine (mea) (4 eq, 100 mmol, 6.11 g) and paraformaldehyde (PFA) (8 eq, 200 mmol,, 6.0 g). All these reactants were reacted together in melt at 85°C and agitated by mechanical stirring for 2 hours to provide the ester-containing benzoxazine monomer named PEGₙ-DPA-mea. The reaction product was used without further purifications for the elaboration of vitrimer materials.

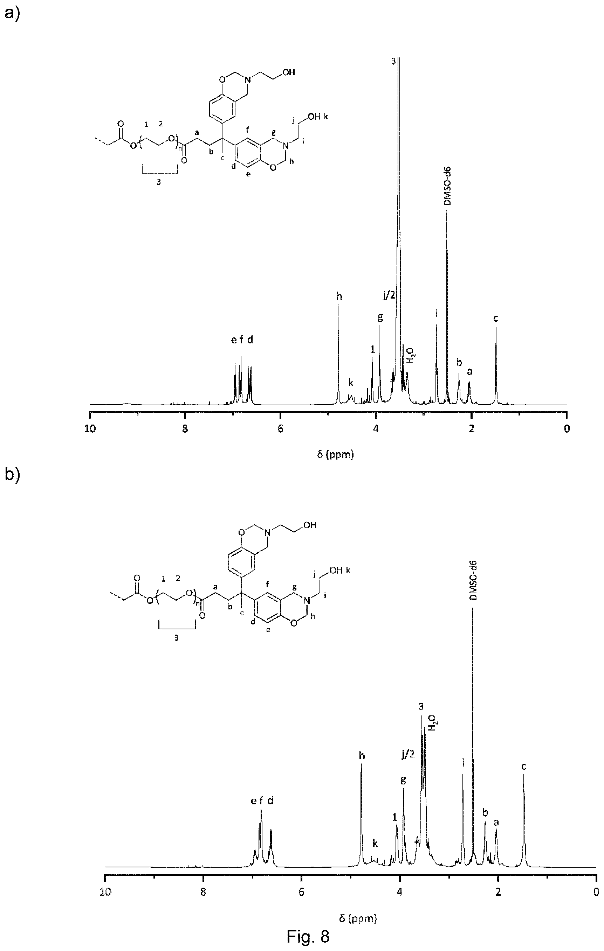

The Figure 8a) and Figure 8b) are respectively displaying the NMR spectrum (AVANCE III HD Bruker spectrometer) of PEG₂₀₀-DPA-mea and PEG₂₀₀₀-DPA-mea ester-containing benzoxazine monomers.

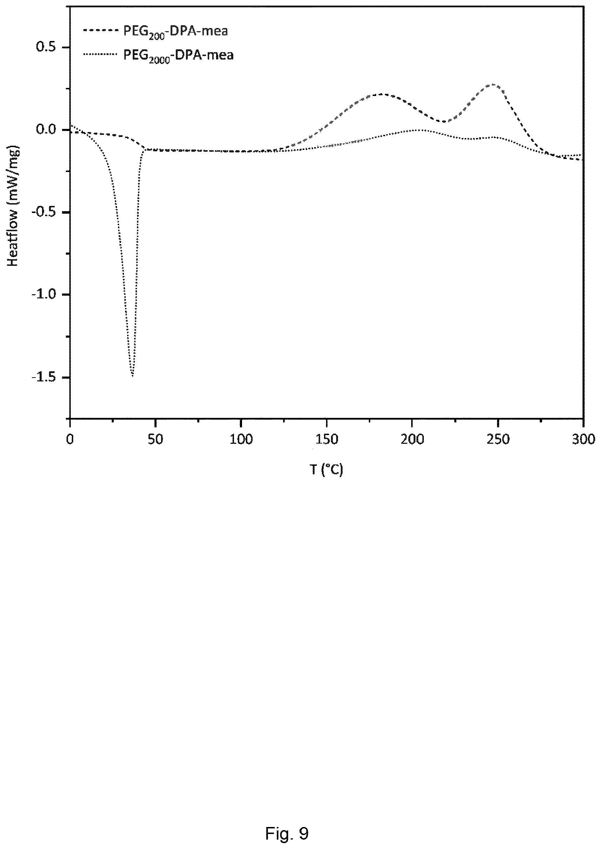

Figure 9 is displaying the DSC curves of the PEG₂₀₀-DPA-mea and PEG₂₀₀₀-DPA-mea monomers. Conditions: 10°C.min⁻¹, N₂ atmosphere (Netzsch DSC 204 F1 Phoenix apparatus). The DSC curve shows an exothermic peak starting at a temperature of 105 and 120°C for PEG₂₀₀-DPA-mea and PEG₂₀₀₀-DPA-mea, respectively. This peak corresponds to the ring opening of the benzoxazine rings upon heating. The second peak corresponds to the thermal decomposition of the ester linkage.

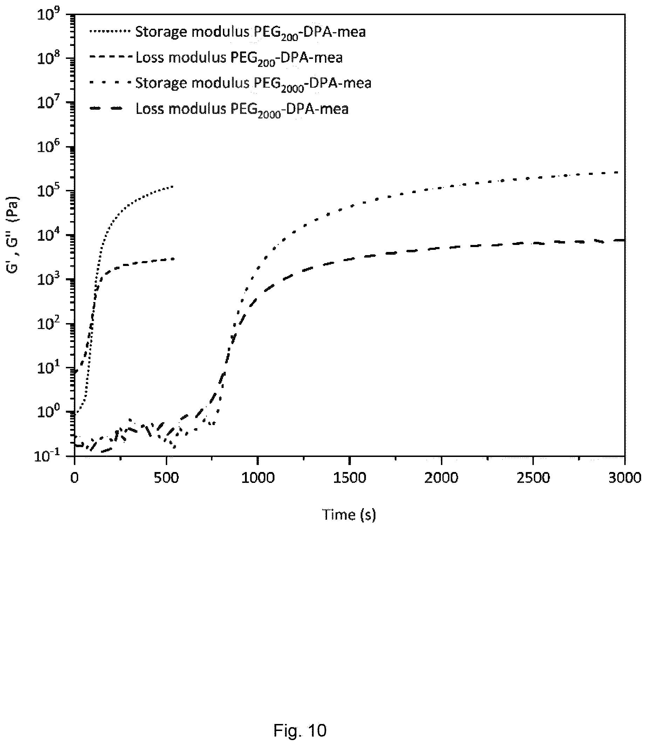

The curing of the PEGₙ-DPA-mea ester-containing benzoxazine monomers was monitored by rheological measurement depicted in Figure 10, to assess the mechanical behaviour of said monomers during the curing process.

The rheogram is performed under the following conditions: 1 Hz, with linear amplitude from 1 to 0.1%; 25 mm plates. The test is performed following a heating ramp from 80°C to 140°C at 15°C/min followed by an isothermal measurement at 140°C. The storage and loss modulus are recorded as a function of time. The term "gelation time" is defined as the time when the storage and the loss modulus of the soften monomer increases abruptly to transform into a gel. The gelation is defined by the crossover point between the storage and the loss modulus. At 140°C, the gelation time is reached after 116 s and 864 s, respectively for PEG₂₀₀ and PEG₂₀₀₀.

### Example 4: synthesis of a vitrimer obtained through the curing of PEGₙ-DPA-mea ester-containing benzoxazine monomers

The benzoxazine monomers obtained in Example 3 was polymerized in a Teflon mold at 150°C during 1h for the obtention of a PEGₙ-DPA-mea derivatives polybenzoxazine vitrimer material (n = 200 or 2000).

Swelling experiments were performed in water to assess the formation of a cross-linked network of the vitrimer obtained through the curing of the PEGₙ-DPA-mea monomer. Vitrimers samples swell of 10% and 200%, respectively for PEG₂₀₀ and PEG₂₀₀₀.

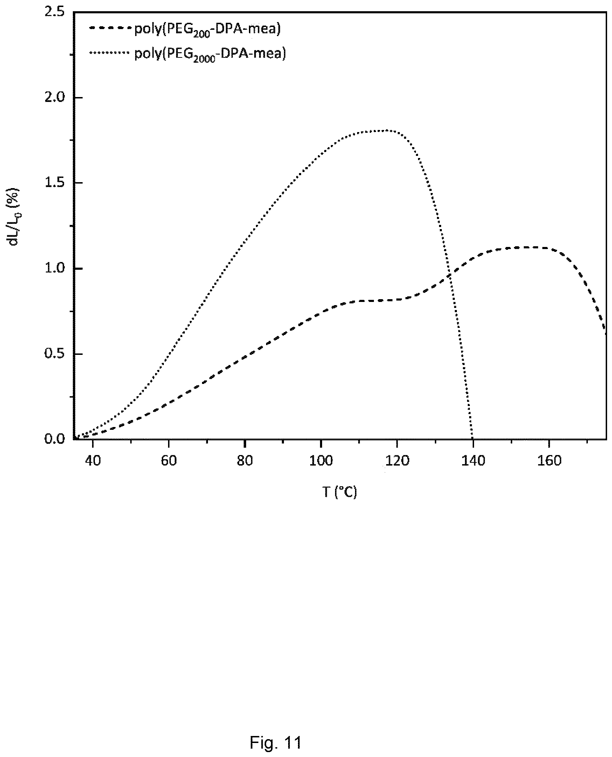

Dilatometry thermograms of the vitrimer samples are reported in Figure 11. The device used is the Netzsch DIL 402 C apparatus with experimental conditions of 2°C.min⁻¹, N₂ atmosphere. The first plateau corresponds to the T_{g} of material while the second is characteristic of the glass-like nature of the Tᵥ.

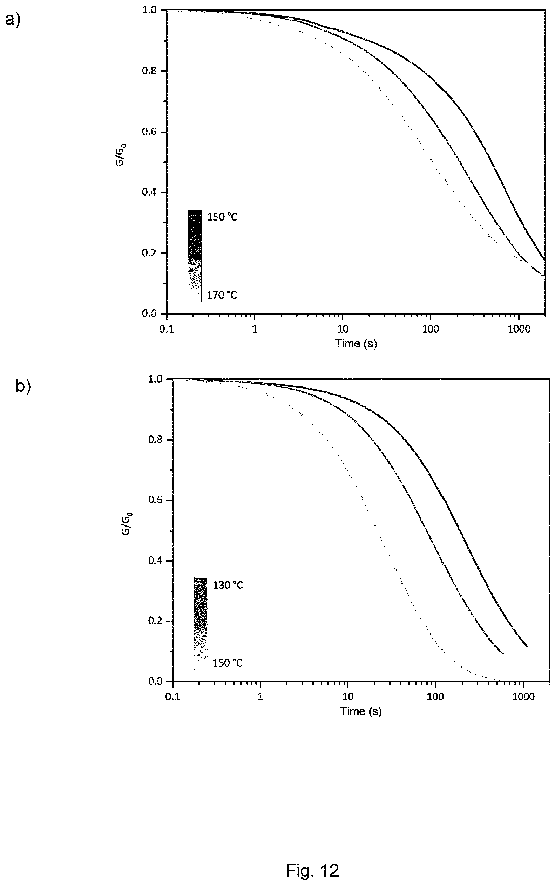

Viscoelastic properties of poly(PEGₙ-DPA-mea) vitrimer were studied by stress relaxation experiments (Figure 12a): poly(PEG₂₀₀-DPA-mea) vitrimer and Figure 12b): poly(PEG₂₀₀₀-DPA-mea) vitrimer). The relaxation time of the polymer was clearly noticeable and proportionally decreasing upon heating from 150°C (814 s) to 170°C (208 s) for PEG₂₀₀-DPA-mea and from 130°C (315 s) to 150°C (36 s) for PEG₂₀₀₀-DPA-mea.

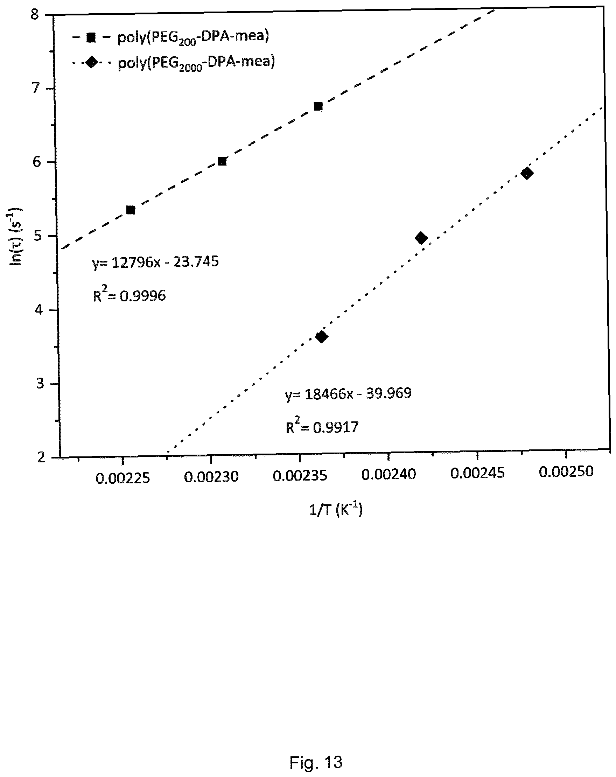

The temperature dependence of the relaxation time was plotted following the Arrhenius law in Figure 13. The trend line fits to a thermally activated behaviour for the relaxation time. The high correlation coefficient (R²= 0.9996 and 0.9817 respectively for PEG₂₀₀ and PEG₂₀₀₀) means that these data are perfectly fitting the Arrhenius law. The activation energy from the Arrhenius equation was extracted using the slope of the trend line. The activation energy obtained from stress relaxation is 106 and 154 kJ.mol⁻¹ respectively for poly(PEG₂₀₀-DPA-mea) and poly(PEG₂₀₀₀-DPA-mea) vitrimer.

### Example 5: synthesis of a benzoxazine monomer from phloretic acid as a phenolic acid derivative

The first step, step a), corresponds to a Fischer esterification between polyethylene glycol (PEG₄₀₀) (Mₙ = 400 g.mol⁻¹ , p = 8-9, 1 eq, 10 g) and phloretic acid (PA) (2 eq, 8.31 g) in presence of p-toluene sulfonic acid (pTSA) introduced in catalytic amount (1 wt%). PEG₄₀₀, PA and pTSA were reacted together in melt at 110°C and agitated by mechanical stirring for 24 hours, to provide phloretic acid terminated polyethylene glycol (PEG₄₀₀-DPA).

The second step, step b), corresponds to a Mannich condensation between phloretic acid terminated polyethylene glycol (PEG₄₀₀-PA) (1 eq, 17.3 g), ethanolamine (mea) (2 eq, 3.04 g) and paraformaldehyde (PFA) (4 eq, 2.98 g). All these reactants were reacted together in melt at 85°C and agitated by mechanical stirring for 2 hours to provide the ester-containing benzoxazine monomer named PEG₄₀₀-PA-mea. The reaction product was used without further purifications for the elaboration of vitrimer materials.

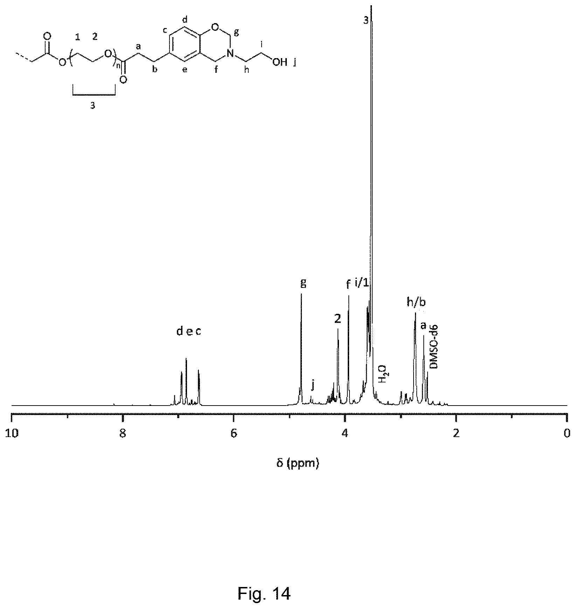

Figure 14 is displaying the NMR spectrum (AVANCE III HD Bruker spectrometer) of PEG₄₀₀-PA-mea ester-containing benzoxazine monomer.

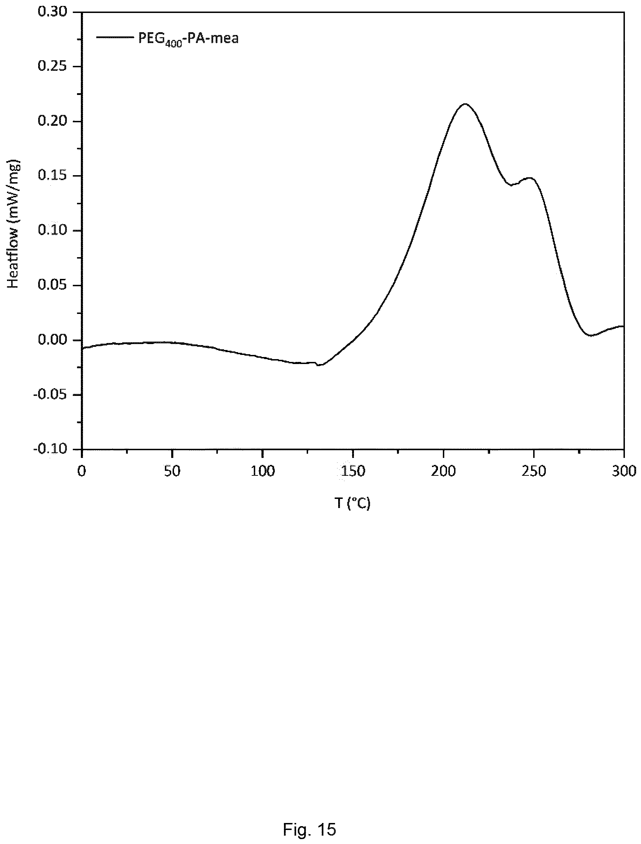

Figure 15 is displaying the DSC curve of the PEG₄₀₀-PA-mea monomer. Conditions: 10°C.min⁻¹, N₂ atmosphere (Netzsch DSC 204 F1 Phoenix apparatus). The DSC curve shows an exothermic peak starting at a temperature of 132°C for PEG₄₀₀-PA-mea. This peak corresponds to the ring opening of the benzoxazine rings upon heating. The second peak corresponds to the thermal decomposition of the ester linkage.

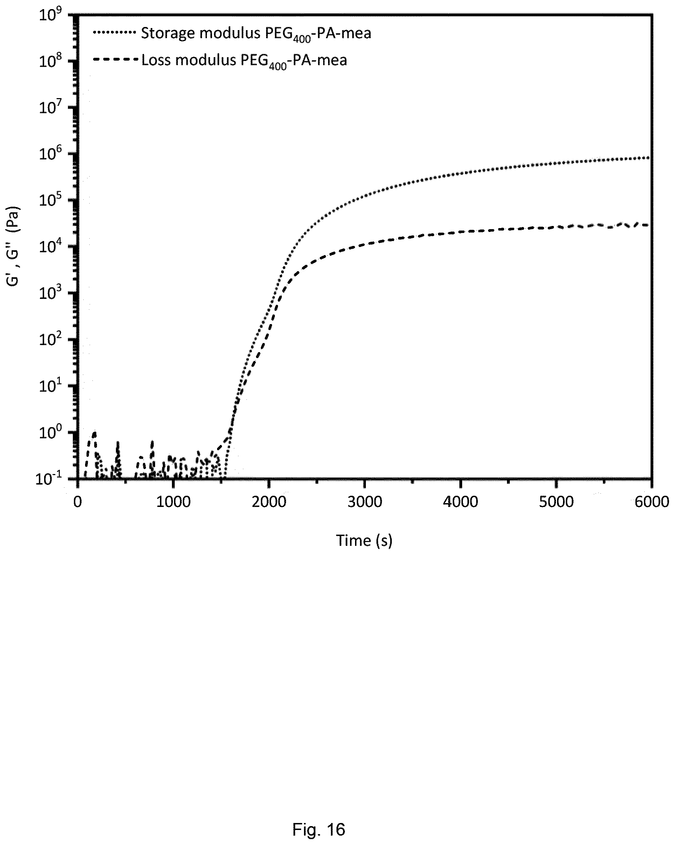

The curing of the PEG₄₀₀-PA-mea ester-containing benzoxazine monomer was monitored by rheological measurement in Figure 16, to assess the mechanical behaviour of said monomers during the curing process.

The rheogram is performed under the following conditions: 1 Hz, with linear amplitude from 1 to 0.1%; 25 mm plates. The test is performed following a heating ramp from 80°C to 140°C at 15°C/min followed by an isothermal measurement at 140°C. The storage and loss modulus are recorded as a function of time. The term "gelation time" is defined as the time when the storage and the loss modulus of the soften monomer increases abruptly to transform into a gel. The gelation is defined by the crossover point between the storage and the loss modulus. At 140°C, the gelation time is reached after 27 min.

### Example 6: synthesis of a vitrimer obtained through the curing of PEG₄₀₀-PA-mea ester-containing benzoxazine monomers

The benzoxazine monomer obtained in Example 5 was polymerized in a Teflon mold at 150°C during 1h for the obtention of a PEG₄₀₀-PA-mea derivatives polybenzoxazine vitrimer material.

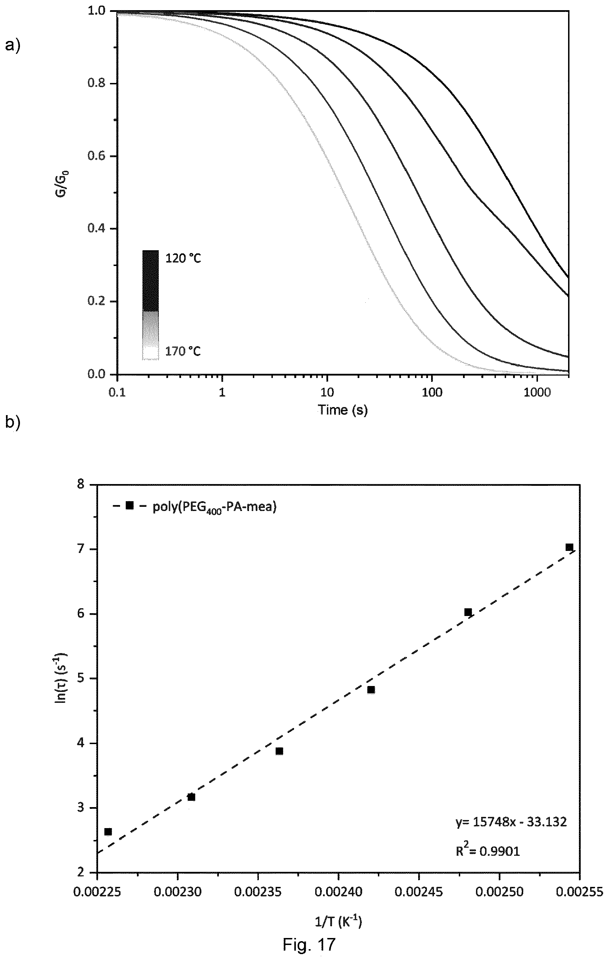

Viscoelastic properties of poly(PEG₄₀₀-PA-mea) vitrimer were studied by stress relaxation experiments (Figure 17a)). The relaxation time of the polymer was clearly noticeable and proportionally decreasing upon heating from 120°C (1131 s) to 170°C (14 s).

The temperature dependence of the relaxation time was plotted following the Arrhenius law (Figure 17b)). The trend line fits to a thermally activated behaviour for the relaxation time. The high correlation coefficient (R²= 0.9901) means that these data are perfectly fitting the Arrhenius law. The activation energy from the Arrhenius equation was extracted using the slope of the trend line. The activation energy obtained from stress relaxation is 131 kJ.mol⁻¹.

## Claims

1. An ester containing benzoxazine monomer of formula (I) 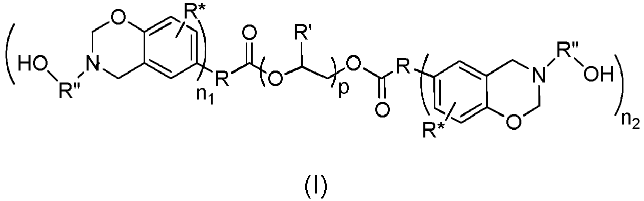 wherein, independently,
at least one R* group is present in the benzoxazine cycle, and is selected from the group consisting of H, an aliphatic C₁-C₆ alkyl group, OH, an aliphatic C₁-C₆ alkoxy group, an aliphatic C₂-C₆ alkenyl group, an aliphatic C₁-C₆ alkyl or alkoxy substituted or unsubstituted phenyl group, 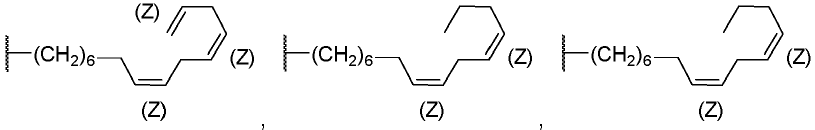 and 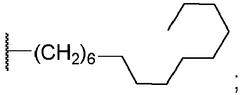
R is either selected from the group consisting of an aliphatic C₁-C₆ alkyl group, an aliphatic C₁-C₆ alkyl or alkoxy substituted or unsubstituted phenyl group, a C₂-C₆ alkenyl group, -(CH₂)ₙ₃- wherein n₃ is an integer from 1 to 10, -CH(aliphatic C₁-C₆ alkyl group), -CH(aliphatic C₁-C₆ alkyl or alkoxy substituted or unsubstituted phenyl group), or R is omitted;
R' is selected from the group consisting of H, -(CH₂)ₙ₃-OH, and 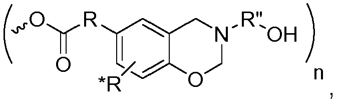 wherein n = n₁ = n₂ and are, independently, an integer of from 1 to 3, and R, R* and n₃ are as defined above;
R" is an aliphatic C₁-C₆ alkyl group; and
p is an integer of from 1 to 50.

2. The ester containing benzoxazine monomer according to claim 1, wherein:
at least one R* group is present in the benzoxazine cycle, more preferably 1-3 R* group(s), and the R* group is selected from the group consisting of H, said H being more preferably in phenolic ortho-position, an aliphatic C₁-C₄ alkyl group, OH, an aliphatic C₁-C₄ alkoxy group, 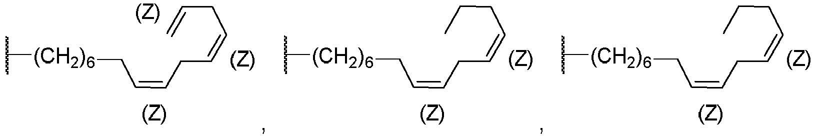 and 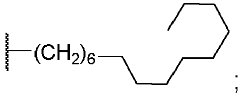
R is either selected from the group consisting of an aliphatic C₁-C₃ alkyl group, an aliphatic C₁-C₃ alkyl or alkoxy substituted or unsubstituted phenyl group, a C₂-C₄ alkenyl group, -(CH₂)ₙ₃- wherein n₃ is an integer from 1 to 6, -CH(aliphatic C₁-C₃ alkyl group), -CH(aliphatic C₁-C₃ alkyl or alkoxy substituted or unsubstituted phenyl group), or R is omitted;
R' is selected from the group consisting of H, -(CH₂)ₙ₃-OH, and 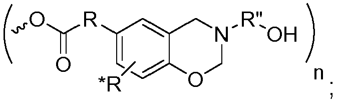 wherein n = n₁ = n₂ and are, independently, an integer of from 1 to 3, more preferably are 1 or 2, and R, the least one R* and n₃ are as defined above, R" is an aliphatic C₁-C₆ alkyl group.

3. A process for synthesizing an ester-containing benzoxazine monomer of formula (I) according to claim 1 or 2, comprising the following steps consisting of:
a) reacting a phenolic acid derivative of formula (II), comprising at least one R* group, 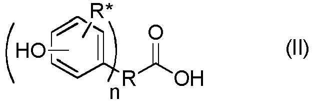 with a polyfunctional molecule or oligomer of formula (III) 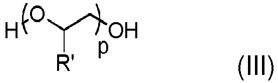 at a temperature of from 25°C to 200°C, during 1h-72h, in the presence of a catalyst of Bronsted acid type, resulting in a phenol terminated oligomer or molecule of formula (IV) 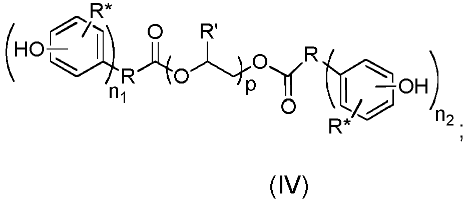 and
b) reacting the compound of formula (IV) with a mixture of:
- an amino-alcohol bifunctional derivative of formula (V): 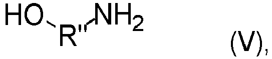 and
- an aldehyde derivative,
at a temperature range of from 25°C to 100°C, during 0,5h to 48h, wherein R, R', R", the at least one R* group, n, n₁, n₂, p are, independently, as defined above, with the proviso that when the at least one R* group of the phenolic acid derivative is in *ortho* position with regard to -OH group, then R* is H.

4. The process according to claim 3, wherein the phenolic acid derivative is selected from the group consisting of mono-, di-, tri-hydroxybenzoic acid derivatives, anacardic acid derivatives, hydroxycinnamic acid derivatives, aliphatic X-hydroxyphenyl acid derivatives, wherein X is 2-4, aliphatic diphenolic acid derivatives and triphenolic acid derivatives, or mixtures thereof.

5. The process according to claim 4, wherein the aliphatic mono-, di-, tri-hydroxybenzoic acid derivatives are of formula (VI) 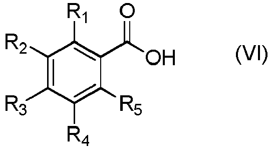 wherein R is omitted, and at least one of R₁ to R₅ corresponds to R*, and at least one among R₁-R₅ is selected from the group consisting of 1, 2 and 3 hydroxyl group(s), then at least one H is in phenolic ortho-position, the rest being at least one of H and an aliphatic alkyl group of C₁-C₆.

6. The process according to claim 4 or 5, wherein the anacardic acid derivatives are of formula (VII), wherein R₆=R*, 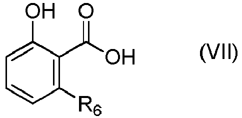 wherein R is omitted, and R₆ is

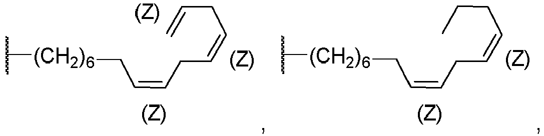

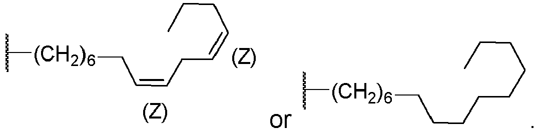

7. The process according to any of claims 4 to 6, wherein the hydroxycinnamic acid derivatives are of formula (VIII) 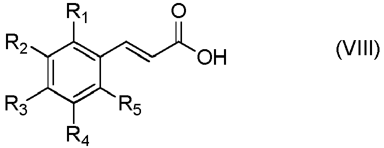 wherein at least one of R₁ to R₅ corresponds to R*, and at least one among R₁-R₅ is selected from the group consisting of 1 and 2 hydroxyl group(s) and at least one H being in phenolic ortho-position, the rest being at least one of H and an aliphatic alkyl or alkoxy group of C₁-C₆.

8. The process according to any of claims 4 to 7, wherein the aliphatic X-hydroxyphenyl acid derivatives are selected from the group consisting of aliphatic di-hydroxyphenyl acids (X=2), aliphatic tri-hydroxyphenyl acids (X=3) and aliphatic tetra-hydroxyphenyl acids (X=4) of formula (IX), or mixtures thereof 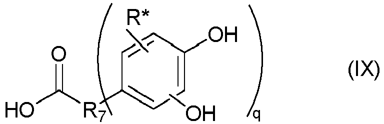 wherein
R₇, corresponding to R, independently of the nature of X-hydroxyphenyl aliphatic acid derivatives, is selected from the group consisting of (CH₂)ₙ₄, CH(CH₂)ₙ₅-(aliphatic C₁-C₆ alkyl or alkoxy substituted or unsubstituted phenyl group), wherein n₄ is an integer from 1 to 12, preferably from 1 to 10, n₅ is an integer from 0 to 12, preferably from 0 to 10, CH(CH₂)ₙ₅(CH₃), CH(CH(CH₃)₂), C(CH₃)₂, CH(aliphatic C₁-C₆ alkyl or alkoxy substituted or unsubstituted phenyl group);
the number of R* in the ring is depending on the number of hydroxyl groups in the ring, and at least one R*, preferably of from 1 to 3, is H towards the phenolic *ortho-*position, and, independently, is selected from the group consisting of (CH₂)ₙ₄CH₃, (CH₂)ₙ₄-(aliphatic C₁-C₆ aliphatic alkyl or alkoxy substituted or unsubstituted phenyl group), wherein n₄ is an integer from 1 to 12, preferably from 1 to 10, more preferably 1-6, and (CH₂)ₙ₄(CH(CH₃)₂); and
the integer q is comprised between 1 and 3.

9. The process according to any of claims 4 to 8, wherein the aliphatic diphenolic acid derivatives are of formula (X) 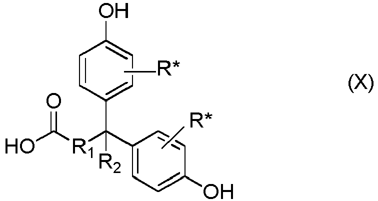 wherein
on each respective phenolic cycle, at least one R*, preferably of from 1 to 3, is H towards the phenolic *ortho*-position, and otherwise R* and R₂, independently, are selected from the group consisting of (CH₂)ₙ₄CH₃, (CH₂)ₙ₄-(aliphatic C₁-C₆ aliphatic alkyl or alkoxy substituted or unsubstituted phenyl group), wherein n₄ is an integer from 1 to 12, preferably from 1 to 10, more preferably from 1 to 6, and (CH₂)ₙ₄(CH(CH₃)₂), and
R₁ is selected from the group consisting of (CH₂)ₙ₅, wherein n₅ is an integer from 1 to 3, CH(CH₂)ₙ₅(CH₃), CH(CH(CH₃)₂) and C(CH₃)₂.

10. The process according to any of claims 3 to 9, wherein the compound of formula (III) has p values of 1-30, better 1-20, especially 1-10, and represents, when R'=H, a polyethylene glycol (PEG) with a molecular weight (MW) in the range of from 4 MW of the C₂H₄O unit to 50 MW of the C₂H₄O unit.

11. The process according to any of claims 3 to 10, wherein the respective stoichiometry of starting reactants on step a), phenolic acid derivative:polyfonctional molecule or oligomer is 1,0-3,0 eq.:1,0 eq., resulting in an 1,0 eq. of phenol terminated oligomer or molecule of formula (IV).

12. The process according to any of claims 3 to 11, wherein the respective stoichiometry of starting reactants on step b), phenol terminated oligomer or molecule: amino-alcohol bifunctional derivative:aldehyde derivative is 1,0 eq.:1,0-18,0 eq.:2,0-36,0 eq., resulting in an 1,0 eq. of the ester-containing benzoxazine monomer.

13. A process for preparing polybenzoxazine derivative vitrimers comprising the step of polymerization of an ester-containing benzoxazine monomer of claim 1 or 2 or as obtainable by the process of any of claims 3-12, at temperatures within the range of from 100°C to 250°C for 1h to 24h.

14. A polybenzoxazine derivative vitrimer, that may be obtained by the process according to claim 13, exhibiting at least one of the following characteristics:
(i) Tᵥ values of from 120°C to 220°C; preferably of from 150°C to 200°C, more preferably of from 150°C to 170°C, and
(ii) Relaxation temperature values, ≥ Tᵥ values, of from 120°C to 270°C, preferably of from 150°C to 200°C, more preferably of from 150°C to 180°C.

15. A polybenzoxazine derivative vitrimer according to claim 14, exhibiting at least one of the following characteristics selected from the group consisting of:
- a relaxation time of from 0,5 s to 2 h, preferably of from 1 s to 1 h, more preferably of from 5 s to 50 min;
- an activation energy related to relaxation times of from 50 kJ/mol to 200 kJ/mol, preferably of from 70 kJ/mol to 170 kJ/mol, more preferably of from 100 kJ/mol to 160 kJ/mol; and
- a processing temperature of from 100°C to 250°C, preferably of from 130°C to 250°C, more preferably of from 150°C to 200°C, most preferably of from 150°C to 170°C.

## Patentansprüche

1. Ein Ester enthaltendes Benzoxazin-Monomer der Formel (I) 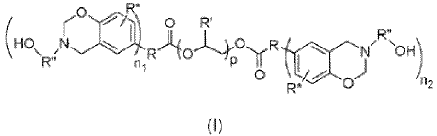 worin unabhängig voneinander
mindestens eine R*-Gruppe in dem Benzoxazin-Zyklus vorhanden ist und ausgewählt ist aus der Gruppe bestehend aus H, einer aliphatischen C₁-C₆-Alkylgruppe, OH, einer aliphatischen C₁₋C₆-Alkoxygruppe, einer aliphatischen C2-C₆-Alkenylgruppe, einer aliphatischen C₁-C₆-Alkyl- oder Alkoxy-substituierten oder unsubstituierten Phenylgruppe, 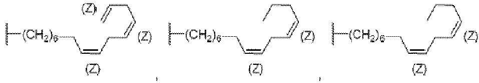 und 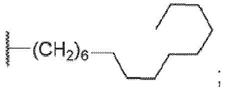
R entweder ausgewählt ist aus der Gruppe bestehend aus einer aliphatischen C₁-C₆-Alkylgruppe, einer aliphatischen C₁₋C₆-Alkyl- oder Alkoxy-substituierten oder unsubstituierten Phenylgruppe, einer C2-C₆-Alkenylgruppe, -(CH₂)ₙ₃-, worin n₃ eine ganze Zahl von 1 bis 10 ist, -CH(aliphatische C₁-C₆-Alkylgruppe), -CH(aliphatische C₁-C₆-Alkyl- oder Alkoxy-substituierte oder unsubstituierte Phenylgruppe), oder R weggelassen wird;
R' ist ausgewählt aus der Gruppe bestehend aus H, -(CH₂)ₙ₃-OH, und 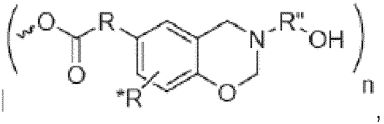 worin n = n₁ = n₂ und unabhängig voneinander eine ganze Zahl von 1 bis 3 sind, und R, R* und n₃ wie oben definiert sind;
R" ist eine aliphatische C₁-C₆-Alkylgruppe; und
p eine ganze Zahl von 1 bis 50 ist.

2. Das esterhaltige Benzoxazin-Monomer nach Anspruch 1, wobei mindestens eine R*-Gruppe im Benzoxazin-Zyklus vorhanden ist, vorzugsweise 1-3 R*-Gruppen, und die R*-Gruppe ausgewählt ist aus der Gruppe bestehend aus H, wobei das H vorzugsweise in phenolischer ortho-Position ist, einer aliphatischen C1-C4-Alkylgruppe, OH, einer aliphatischen C1-C4-Alkoxygruppe, 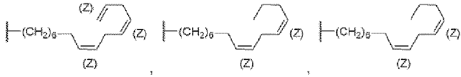 und 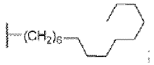
R entweder ausgewählt ist aus der Gruppe bestehend aus einer aliphatischen C₁-C₃-Alkylgruppe, einer aliphatischen C₁-C₃-Alkyl- oder Alkoxy-substituierten oder unsubstituierten Phenylgruppe, einer C₂-C₄-Alkenylgruppe, -(CH₂)ₙ₃-, worin n₃ eine ganze Zahl von 1 bis 6 ist, -CH(aliphatische C₁-C₃-Alkylgruppe), -CH(aliphatische C₁-C₃-Alkyl- oder Alkoxy-substituierte oder unsubstituierte Phenylgruppe), oder R weggelassen ist;
R' ausgewählt ist aus der Gruppe bestehend aus H, -(CH₂)ₙ₃-OH, und 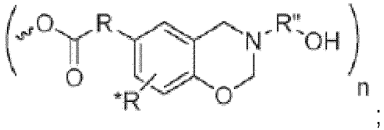 worin n = n₁ = n₂ und unabhängig voneinander eine ganze Zahl von 1 bis 3, vorzugsweise 1 oder 2 sind, und R, das mindestens eine R* und n₃ wie oben definiert sind, R" eine aliphatische C₁-C₆-Alkylgruppe ist.

3. Ein Verfahren zur Synthese eines esterhaltigen Benzoxazin-Monomers der Formel (I) nach Anspruch 1 oder 2, das die folgenden Schritte umfasst, bestehend aus:
a) Umsetzen eines Phenolsäurederivats der Formel (II), das mindestens eine R*-Gruppe enthält, 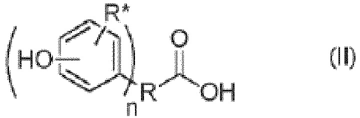 mit einem polyfunktionellen Molekül oder Oligomer der Formel (111) 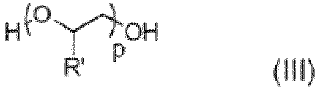 bei einer Temperatur von 25°C bis 200°C während 1h-72h in Gegenwart eines Katalysators des Typs Brönsted-Säure, wodurch ein Oligomer oder Molekül mit Phenol-Endgruppen der Formel (IV) 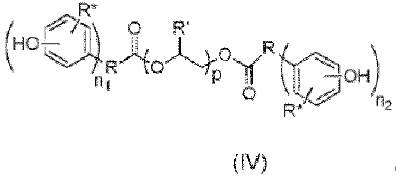 entsteht; und
b) Umsetzen der Verbindung der Formel (IV) mit einer Mischung aus:
- einem bifunktionellen Aminoalkoholderivat der Formel (V): 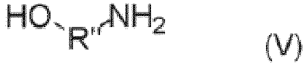 und
- einem Aldehydderivat,
bei einer Temperatur im Bereich von 25°C bis 100°C über einen Zeitraum von 0,5h bis 48h, wobei R, R', R", die mindestens eine R*-Gruppe, n, n₁, n₂, p unabhängig voneinander wie oben definiert sind, mit der Maßgabe, dass, wenn die mindestens eine R*-Gruppe des Phenolsäurederivats in ortho-Position in Bezug auf die -OH-Gruppe steht, R* H ist.

4. Das Verfahren nach Anspruch 3, wobei das Phenolsäurederivat ausgewählt ist aus der Gruppe bestehend aus Mono-, Di-, Tri-Hydroxybenzoesäurederivaten, Anacardsäurederivaten, Hydroxyzimtsäurederivaten, aliphatischen X-Hydroxyphenylsäurederivaten, wobei X 2-4 ist, aliphatischen Diphenolsäurederivaten und Triphenolsäurederivaten oder Mischungen davon.

5. Das Verfahren nach Anspruch 4, wobei die aliphatischen Mono-, Di-, Tri-Hydroxybenzoesäure-Derivate die Formel (VI) 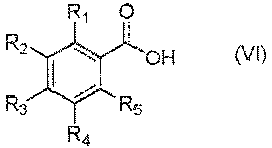 haben, in der R weggelassen ist und mindestens einer der Reste R₁ bis R₅ R* entspricht und mindestens einer der Reste R₁-R₅ aus der Gruppe ausgewählt ist, die aus 1, 2 und 3 Hydroxylgruppen besteht, wobei sich mindestens ein H in phenolischer ortho-Position befindet und der Rest aus mindestens einem der Reste H und einer aliphatischen Alkylgruppe von C₁-C₆ besteht.

6. Das Verfahren nach Anspruch 4 oder 5, wobei die Anacardsäurederivate die Formel (VII) haben, wobei R₆=R*, 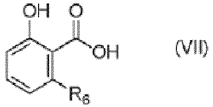 worin R weggelassen ist und R₆ 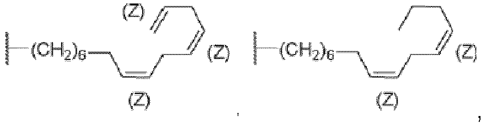 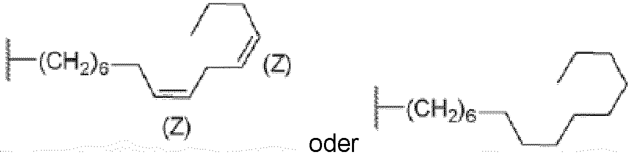 ist.

7. Das Verfahren nach einem der Ansprüche 4 bis 6, wobei die Hydroxyzimtsäurederivate die Formel (VIII) haben 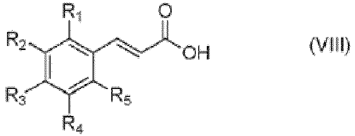 worin mindestens einer der R₁ bis R₅ R* entspricht und mindestens einer der R₁-R₅ aus der Gruppe ausgewählt ist, die aus 1 und 2 Hydroxylgruppen und mindestens einem H in phenolischer *ortho*-Position besteht, wobei der Rest mindestens eines von H und einer aliphatischen Alkyl- oder Alkoxygruppe von C₁-C₆ ist.

8. Das Verfahren nach einem der Ansprüche 4 bis 7, wobei die aliphatischen X-Hydroxyphenylsäurederivate ausgewählt sind aus der Gruppe bestehend aus aliphatischen Di-Hydroxyphenylsäuren (X=2), aliphatischen Tri-Hydroxyphenylsäuren (X=3) und aliphatischen Tetra-Hydroxyphenylsäuren (X=4) der Formel (IX) oder Mischungen davon, 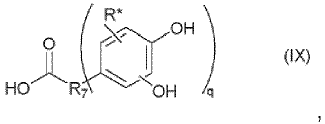 worin
R₇, das R entspricht, unabhängig von der Art der aliphatischen X-Hydroxyphenylsäurederivate, ausgewählt ist aus der Gruppe bestehend aus (CH₂)ₙ₄, CH(CH₂)ₙ₅- (aliphatische C₁-C₆-Alkyl- oder Alkoxy-substituierte oder unsubstituierte Phenylgruppe), worin n₄ eine ganze Zahl von 1 bis 12, vorzugsweise von 1 bis 10 ist, n₅ eine ganze Zahl von 0 bis 12, vorzugsweise von 0 bis 10 ist, CH(CH₂)ₙ₅(CH₃), CH(CH(CH₃)₂), C(CH₃)₂, CH(aliphatische C₁- C₆-Alkyl- oder Alkoxy-substituierte oder unsubstituierte Phenylgruppe);
die Anzahl der R* im Ring ist abhängig von der Anzahl der Hydroxylgruppen im Ring, und mindestens ein R*, vorzugsweise 1 bis 3, ist H in Richtung der phenolischen ortho-Position und ist unabhängig ausgewählt aus der Gruppe bestehend aus (CH₂)ₙ₄CH₃, (CH₂)ₙ₄-(aliphatische C₁-C₆ aliphatische Alkyl- oder Alkoxy-substituierte oder unsubstituierte Phenylgruppe), wobei n₄ eine ganze Zahl von 1 bis 12, vorzugsweise von 1 bis 10, besonders bevorzugt 1-6, ist, und (CH₂)ₙ₄{CH(CH₃)₂); und
die ganze Zahl q zwischen 1 und 3 liegt.

9. Das Verfahren nach einem der Ansprüche 4 bis 8, wobei die aliphatischen Diphenolsäurederivate die Formel (X) 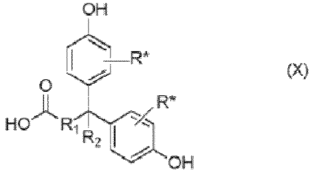 haben, worin
an jedem jeweiligen phenolischen Zyklus mindestens ein R*, vorzugsweise von 1 bis 3, H in Richtung der phenolischen ortho-Position ist, und ansonsten R* und R₂ unabhängig voneinander ausgewählt sind aus der Gruppe bestehend aus (CH₂)ₙ₄CH₃, (CH₂)ₙ₄-(aliphatisches C₁-C₆ aliphatisches Alkyl oder Alkoxy substituierte oder unsubstituierte Phenylgruppe), worin n₄ eine ganze Zahl von 1 bis 12, vorzugsweise von 1 bis 10, besonders bevorzugt von 1 bis 6 ist, und (CH₂)ₙ₄(CH(CH₃)₂), und
R₁ ausgewählt ist aus der Gruppe bestehend aus (CH₂)ₙ₅, wobei n₅ eine ganze Zahl von 1 bis 3 ist, CH(CH₂)ₙ₅(CH₃), CH(CH(CH₃)₂) und C(CH₃)₂.

10. Das Verfahren nach einem der Ansprüche 3 bis 9, wobei die Verbindung der Formel (III) p-Werte von 1-30, besser 1-20, insbesondere 1-10, aufweist und, wenn R'=H, ein Polyethylenglykol (PEG) mit einem Molekulargewicht (MW) im Bereich von 4 MW der C₂H₄O-Einheit bis 50 MW der C₂H₄O-Einheit darstellt.

11. Das Verfahren nach einem der Ansprüche 3 bis 10, wobei die jeweilige Stöchiometrie der Ausgangsreaktanten in Schritt a), Phenolsäurederivat: polyfunktionelles Molekül oder Oligomer, 1,0-3,0 Äquivalente:1,0 Äquivalente beträgt, was zu einem 1,0 Äquivalente Phenol-terminierten Oligomer oder Molekül der Formel (IV) führt.

12. Das Verfahren nach einem der Ansprüche 3 bis 11, wobei die jeweilige Stöchiometrie der Ausgangsreaktanten in Schritt b), Phenol-terminiertes Oligomer oder Molekül: bifunktionelles Aminoalkoholderivat:Aldehydderivat, 1,0 Äquivalente:1,0-18,0 Äquivalente:2,0-36,0 Äquivalente beträgt, was zu 1,0 Äquivalenten des esterhaltigen Benzoxazinmonomers führt.

13. Ein Verfahren zur Herstellung von Polybenzoxazinderivat-Vitrimeren, umfassend den Schritt der Polymerisation eines esterhaltigen Benzoxazinmonomers nach Anspruch 1 oder 2 oder wie durch das Verfahren nach einem der Ansprüche 3-12 erhältlich, bei Temperaturen im Bereich von 100°C bis 250°C für 1h bis 24h.

14. Ein Polybenzoxazin-Derivat-Vitrimer, das durch das Verfahren nach Anspruch 13 erhalten werden kann, das mindestens eine der folgenden Eigenschaften aufweist:
(i) Tᵥ-Werte von 120°C bis 220°C; vorzugsweise von 150°C bis 200°C, noch bevorzugter von 150°C bis 170°C, und
(ii) Relaxationstemperaturwerte, ≥ Tᵥ-Werte, von 120°C bis 270°C, vorzugsweise von 150°C bis 200°C, noch bevorzugter von 150°C bis 180°C.

15. Ein Polybenzoxazinderivat-Vitrimer nach Anspruch 14, das mindestens eine der folgenden Eigenschaften aufweist, ausgewählt aus der Gruppe bestehend aus:
- einer Relaxationszeit von 0,5 s bis 2 h, vorzugsweise von 1 s bis 1 h, besonders bevorzugt von 5 s bis 50 min;
- einer auf die Relaxationszeiten bezogenen Aktivierungsenergie von 50 kJ/mol bis 200 kJ/mol, vorzugsweise von 70 kJ/mol bis 170 kJ/mol, besonders bevorzugt von 100 kJ/mol bis 160 kJ/mol; und
- eine Verarbeitungstemperatur von 100°C bis 250°C, vorzugsweise von 130°C bis 250°C, noch bevorzugter von 150°C bis 200°C, am meisten bevorzugt von 150°C bis 170°C.

## Revendications

1. Un monomère de benzoxazine contenant un ester ayant la formule (I) 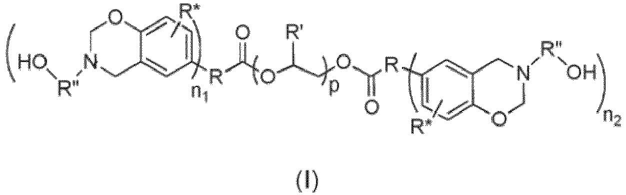 dans lequel, indépendamment,
au moins un groupe R* est présent dans le cycle benzoxazine, et est sélectionné dans le groupement constitué de H, un groupement alkyle aliphatique en C₁-C₆, OH, un groupement alcoxy aliphatique en C₁-C₆, un groupement alcényle aliphatique en C₂-C₆, un groupement phényle aliphatique substitué ou non par un alkyle ou alcoxy en C₁-C₆, 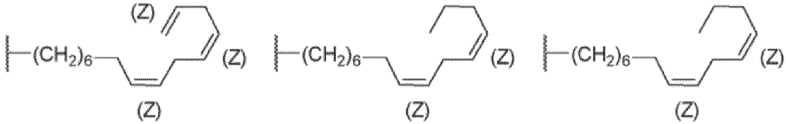 et 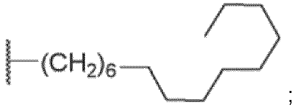
R est sélectionné dans le groupement constitué d'un groupement alkyle aliphatique en C₁-C₆, d'un groupement phényle aliphatique substitué ou non par un alkyle ou alcoxy en C₁-C₆, d'un groupement alcényle en C₂-C₆, -(CH₂)ₙ₃- où n₃ est un nombre entier compris entre 1 et 10, -CH(groupement alkyle aliphatique en C₁-C₆), - CH(groupement phényle aliphatique substitué ou non par un alkyle ou alcoxy en C₁-C₆), ou R est omis ;
R' est sélectionné dans le groupement constitué de H, -(CH₂)ₙ₃-OH, et 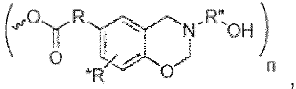 où n = n₁ = n₂ et sont indépendamment, un nombre entier compris entre 1 et 3, et R, R* et n₃ sont tels que définis ci-dessus ;
R" est un groupement alkyle aliphatique en C₁-C₆ ; et
p est un nombre entier compris entre 1 et 50.

2. Le monomère de benzoxazine contenant un ester selon la revendication 1, dans lequel :
au moins un groupement R* est présent dans le cycle benzoxazine, plus préférentiellement 1 à 3 groupement(s) R*, et le groupement R* est sélectionné dans le groupement constitué de H, ledit H étant plus préférentiellement en *ortho*-position phénolique, un groupement alkyle aliphatique en C₁-C₄, OH, un groupement alcoxy aliphatique en C₁-C_{4,} 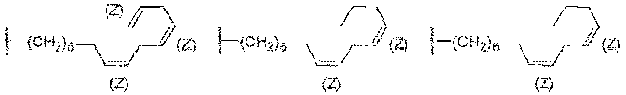 et 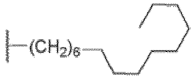
R est sélectionné dans le groupement constitué d'un groupement alkyle aliphatique en C₁-C₃, d'un groupement phényle aliphatique substitué ou non par un alkyle ou alcoxy en C₁-C₃, d'un groupement alcényle en C₂-C₄, -(CH₂)ₙ₃- où n₃ est un nombre entier compris entre 1 et 6, -CH(groupement alkyle aliphatique en C₁-C₃), - CH(groupement phényle aliphatique substitué ou non par un alkyle ou alcoxy en C₁-C₃), ou R est omis ;
R' est sélectionné dans le groupement constitué de H, -(CH₂)ₙ₃-OH, et 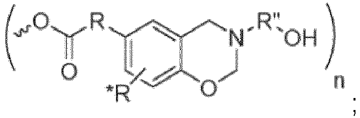
où n = n₁ = n₂ et sont, indépendamment, un nombre entier compris entre 1 et 3, de préférence 1 ou 2, et R, les au moins R* et n₃ sont tels que définis ci-dessus, R" est un groupement alkyle aliphatique en C₁-C₆.

3. Un procédé de synthèse d'un monomère de benzoxazine contenant un ester ayant la formule (I) selon la revendication 1 ou 2, comprenant les étapes suivantes consistant à :
a) faire réagir un dérivé d'acide phénolique ayant la formule (II), comprenant au moins un groupement R*, 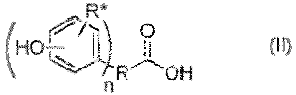 avec une molécule polyfonctionnelle ou un oliomère ayant la formule (III) 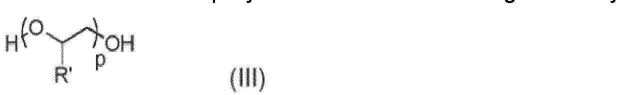 à une température comprise entre 25°C et 200°C, pendant 1h à 72h, en présence d'un catalyseur de type acide de Bronsted, ce qui donne un oligomère ou une molécule à terminaison phénolique ayant la formule (IV) 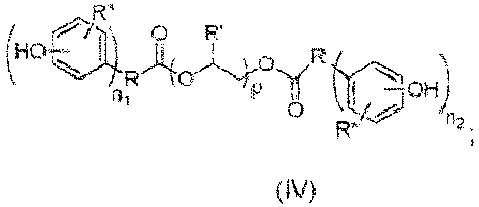 et
b) faire réagir le composé ayant la formule (IV) avec un mélange composé :
- d'un dérivé amino-alcool bifonctionnel ayant la formule (V) : 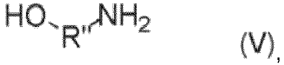 et
- d'un dérivé d'aldéhyde,
à une température comprise entre 25°C et 100°C, pendant 0,5h à 48h, où R, R', R", ledit au moins un groupement R*, n, n₁, n₂, p sont, indépendamment, tels que définis ci-dessus, à condition que lorsque le groupement R* au moins du dérivé d'acide phénolique est en *ortho*-position par rapport au groupement -OH, R* est H.

4. Le procédé selon la revendication 3, dans lequel le dérivé d'acide phénolique est sélectionné dans le groupement constitué des dérivés de l'acide mono-, di-, tri-hydroxybenzoïque, des dérivés de l'acide anacardique, des dérivés de l'acide hydroxycinnamique, des dérivés de l'acide X-hydroxyphényl aliphatique, dans lequel X est compris entre 2 et 4, des dérivés aliphatiques de l'acide diphénolique et des dérivés de l'acide triphénolique, ou des mélanges de ceux-ci.

5. Le procédé selon la revendication 4, dans lequel les dérivés aliphatiques de l'acide mono-, di-, tri-hydroxybenzoïque ont la formule (VI) 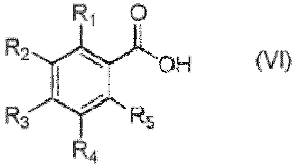 où R est omis, et au moins un de R₁ à R₅ correspond à R*, et au moins un parmi R₁-R₅ est sélectionné dans le groupement constitué de 1, 2 et 3 groupement(s) hydroxyle(s), alors au moins un H est en *ortho*-position phénolique, le reste étant au moins l'un de H et un groupement alkyle aliphatique en C₁-C₆.

6. Le procédé selon la revendication 4 ou 5, dans lequel les dérivés de l'acide anacardique ont la formule (VII), où R₆=R*, 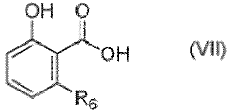 où R est omis, et R₆ est

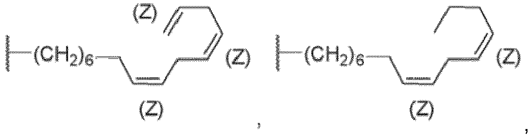

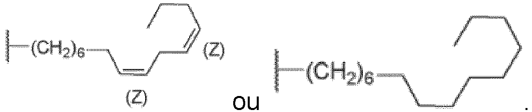

7. Le procédé selon l'une quelconque des revendications 4 à 6, dans lequel les dérivés de l'acide hydroxycinnamique ont la formule (VIII) 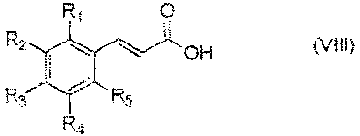 où au moins un de R₁ à R₅ correspond à R*, et au moins un parmi R₁-R₅ est sélectionné dans le groupement constitué de 1 et 2 groupement(s) hydroxyle(s) et au moins un H étant en ortho-position phénolique, le reste étant au moins l'un de H et un groupement alkyle ou alcoxy aliphatique en C₁-C₆.

8. Le procédé selon l'une quelconque des revendications 4 à 7, dans lequel les dérivés de l'acide X-hydroxyphényle aliphatique sont sélectionnés dans le groupement constitué d'acides di-hydroxyphényles aliphatiques (X=2), d'acides tri-hydroxyphényles aliphatiques (X=3) et d'acides tétra-hydroxyphényles aliphatiques (X=4) ayant la formule (IX), ou leurs mélanges où 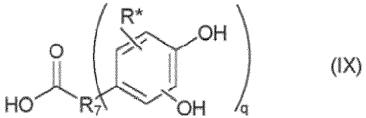
R₇, correspondant à R, indépendamment de la nature des dérivés de l'acide X-hydroxyphényl aliphatique, est sélectionné dans le groupement constitué de (CH₂)ₙ₄, CH(CH₂)ₙ₅- (groupement phényle aliphatique substitué ou non par un alkyle ou alcoxy en C₁-C₆), où n₄ est un nombre entier compris entre 1 et 12, de préférence entre 1 et 10, n₅ est un nombre entier compris entre 0 et 12, de préférence entre 0 et 10, CH(CH₂)ₙ₅(CH₃), CH(CH(CH₃)₂), C(CH₃)₂, CH(groupement phényle aliphatique substitué ou non par un alkyle ou alcoxy en C₁-C₆) ;
le nombre de R* dans l'anneau dépend du nombre de groupements hydroxyle dans l'anneau, et au moins un R*, de préférence de 1 à 3, est H vers l'ortho-position phénolique, et, indépendamment, est sélectionné dans le groupement constitué de (CH₂)ₙ₄CH₃, (CH₂)ₙ₄-(groupement phényle aliphatique substitué ou non par un alkyle ou alcoxy en C₁-C₆), où n₄ est un nombre entier compris entre 1 et 12, de préférence entre 1 et 10, plus préférentiellement entre 1 et 6, et (CH₂)ₙ₄(CH(CH₃)₂) ; et
le nombre entier q est compris entre 1 et 3.

9. Le procédé selon l'une quelconque des revendications 4 à 8, dans lequel les dérivés d'acides diphénoliques aliphatiques ont la formule (X) 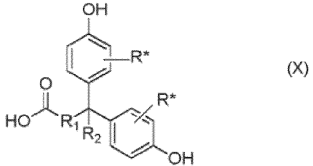 dans laquelle
sur chaque cycle phénolique respectif, au moins un R*, de préférence de 1 à 3, est H vers l'ortho-position phénolique, et autrement R* et R₂, indépendamment, sont sélectionnés dans le groupement constitué de (CH₂)ₙ₄CH₃, (CH₂)ₙ₄-(groupement phényle aliphatique substitué ou non par un alkyle ou alcoxy en C₁-C₆), où n₄ est un nombre entier compris entre 1 et 12, de préférence entre 1 et 10, plus préférentiellement entre 1 et 6, et (CH₂)ₙ₄(CH(CH₃)₂), et
R₁ est sélectionné dans le groupement constitué de (CH₂)ₙ₅, où n₅ est un nombre entier compris entre 1 et 3, CH(CH₂)ₙ₅(CH₃), CH(CH(CH₃)₂) et C(CH₃)₂.

10. Le procédé selon l'une quelconque des revendications 3 à 9, dans lequel le composé ayant la formule (III) a des valeurs p comprises entre 1 et 30, de préférence entre 1 et 20, plus préférentiellement entre 1 et 10, et représente, lorsque R'=H, un polyéthylène glycol (PEG) dont le poids moléculaire (MW) est compris entre 4 MW de l'unité C₂H₄O et 50 MW de l'unité C₂H₄O.

11. Le procédé selon l'une quelconque des revendications 3 à 10, dans lequel la stoechiométrie respective des réactifs de départ à l'étape a), dérivé d'acide phénolique : molécule polyfonctionnelle ou oligomère, est de 1,0 à 3,0 éq. pour 1,0 éq., ce qui donne 1,0 eq d'oligomère ou de molécule à terminaison phénolique ayant la formule (IV).

12. Le procédé selon l'une quelconque des revendications 3 à 11, dans lequel la stoechiométrie respective des réactifs de départ à l'étape b), oligomère ou molécule à terminaison phénolique : dérivé amino-alcool bifonctionnel : dérivé aldéhydique est de 1,0 éq. pour 1,0 à 18,0 éq. : 2,0 à 36,0 éq., ce qui donne 1,0 éq. de monomère de benzoxazine contenant un ester.

13. Un procédé de préparation de vitrimères de dérivés polybenzoxazine comprenant l'étape de polymérisation d'un monomère de benzoxazine contenant un ester selon la revendication 1 ou 2 ou tel qu'il peut être obtenu par le procédé selon l'une des revendications 3 à 12, à des températures comprises entre 100°C et 250°C pendant 1 h à 24 h.

14. Un vitrimère dérivé de la polybenzoxazine, qui peut être obtenu par le procédé selon la revendication 13, présentant au moins l'une des caractéristiques suivantes :
(i) des valeurs de température Tᵥ comprises entre 120°C et 220°C ; de préférence entre 150°C et 200°C, plus préférablement entre 150°C et 170°C, et
(ii) des valeurs de température de relaxation, ≥ valeurs de température Tᵥ, comprises entre 120°C et 270°C, de préférence entre 150°C et 200°C, plus préférablement entre 150°C et 180°C.

15. Un vitrimère dérivé de la polybenzoxazine selon la revendication 14, présentant au moins l'une des caractéristiques suivantes, sélectionnées dans le groupement constitué :
- d'un temps de relaxation compris entre 0,5 s et 2 h, de préférence entre 1 s et 1 h, plus préférablement entre 5 s et 50 min ;
- d'une énergie d'activation liée aux temps de relaxation compris entre 50 kJ/mol et 200 kJ/mol, de préférence entre 70 kJ/mol et 170 kJ/mol, plus préférablement entre 100 kJ/mol et 160 kJ/mol ; et
- une température de traitement comprise entre 100°C et 250°C, de préférence entre 130°C et 250°C, plus préférablement entre 150°C et 200°C, de manière davantage préférée entre 150°C et 170°C.
